# EUROPEAN PATENT SPECIFICATION

(11) **EP 3 491 390 B1**
(45) Date of publication and mention of the grant of the patent: **02.07.2025**
(21) Application number: 17833110.4
(22) Date of filing: 28.07.2017
(51) Int. Cl.: G01N 33/68, G01N 33/53, G01N 33/569

(54) **ESTIMATING CELLULAR POPULATIONS**
SCHÄTZUNG VON ZELLPOPULATIONEN
ESTIMATION DE POPULATIONS CELLULAIRES

(30) Priority: 28.07.2016 AU 2016902981
(43) Date of publication of application: 05.06.2019
(73) Proprietor: Macfarlane Burnet Institute for Medical Research and Public Health Limited, Melbourne, Victoria 3004 (AU)
(72) Inventor: ANDERSON, David, Melbourne Victoria 3004 (AU); PALCHAUDHURI, Riya, Melbourne Victoria 3004 (AU); CROWE, Suzanne, Melbourne Victoria 3004 (AU); PALMER, Clovis, Melbourne Victoria 3004 (AU)
(74) Representative: Dehns
(86) International application number: PCT/AU2017/050788
(87) International publication number: WO 2018/018095

(56) References cited:
- WO-A1-2013/112216
- WO-A1-2016/057945
- WO-A2-2009/043057
- WO-A2-2013/107826
- US-A1- 2014 170 678
- ANONYMOUS: "Sialyl-Lewis X - Wikipedia", 20 November 2023 (2023-11-20), XP093155806, Retrieved from the Internet <URL:https://en.wikipedia.org/wiki/Sialyl-Lewis_X>
- HOFFMAN, J.J.M.L.: "Neutrophil CD 64 as a sepsis biomarker", BIOCHEMIA MEDICA, vol. 21, no. 3, 2011, pages 282 - 290, XP055588989
- RUNDSTROM, G. ET AL.: "Lateral flow immunoassay using europium (III) chelate microparticles and time-resolved fluorescence for eosinophils and neutrophils in whole blood", CLINICAL CHEMISTRY, vol. 53, no. 2, 2007, pages 342 - 348, XP055460839
- MULLER KOBOLD, A.C. ET AL.: "Leukocyte activation in sepsis; correlations with disease state and mortality", INTENSIVE CARE MEDICINE, vol. 26, no. 7, 2000, pages 883 - 892, XP002375269
- LARD, L.R. ET AL.: "Neutrophil activation in sickle cell disease", JOURNAL OF LEUKOCYTE BIOLOGY, vol. 66, no. 3, 1999, pages 411 - 415, XP055460840
- HASSAN, U. ET AL.: "A point-of-care microfluidic biochip for quantification of CD 64 expression from whole blood for sepsis stratification", NATURE COMMUNICATIONS, vol. 8, 3 July 2017 (2017-07-03), pages 15949, XP055460842

## Description

### FIELD

The field of the specification is broadly diagnostics and estimating biomarker expression levels of cellular biological populations. More specifically, assays are provided for identifying or monitoring neutrophil activation, pathophysiological conditions following neutrophil activation and particularly sepsis or severe infection or a risk of developing sepsis or a severe infection in subjects, including neonatal subjects. The present assays are applicable in a wide range of immunoassay formats and developed for algorithm-based diagnostic assays ranging from rapid point of care assays and devices to more data-rich applications employing hard ware and software to input and process data including via an algorithm to assess the statistical significance of altered biomarker levels and formulating output data capable of integrating with pathology platform systems.

### BACKGROUND

Bibliographic details of references in the subject specification are also listed at the end of the specification.

Reference to any prior art in this specification is not, and should not be taken as, acknowledgement or any form of suggestion that this prior art forms part of the common general knowledge in any country.

A diverse range of techniques are used in research, analysis, development and clinically to detect cells of interest. Manual or automated techniques are available to count cells in specially designed chambers that permit cell numbers in a sample to be evaluated. Cells may be stained with particular stains in order to differentiate between cell types. Histochemical techniques may be applied to further differentiate between cells in a sample. The ability of cells to respond to particular antigens by proliferating or producing cytokines, bind other cells, engulf other cells or move by chemotaxis may also be diagnostic. Cell surface markers are particularly useful for differentiating between cell types and evaluating the number of particular cells in a sample and changes in the biological state of such cells in a sample. Many techniques use antibodies to detect the presence of the marker.

Flow cytometry is a powerful tool for identifying and enumerating cells. The flow cytometer detects and counts individual cells passing in a stream through a laser beam. By examining large numbers of cells, flow cytometry can give quantitative data on the percentage of cells bearing different molecules, such as surface immunoglobulin, which characterizes B cells, the T-cell receptor-associated molecules known as CD3, and the CD4 and CD8 co-receptor proteins that distinguish the major T-cell subsets. Individual cells within a mixed population are tagged with specific antibodies labelled with fluorescent dyes, or for example, by specific antibodies followed by labelled anti-immunoglobulin antibodies. The suspended mixture of labelled cells is then forced through an aperture, creating a fine stream of liquid containing cells spaced singly at intervals. As each cell passes through a laser beam it scatters the laser light, and any dye molecules bound to the cell will be excited and will fluoresce. Sensitive photomultiplier tubes detect both the scattered light, which gives information on the size and granularity of the cell, and the fluorescence emissions, which give information on the binding of the labelled antibodies and hence on the expression of cell-surface proteins by each cell. If two or more antibodies are used, each coupled to a different fluorescent dye, then the data may be displayed in the form of a two-dimensional scatter diagram or as a contour diagram, where the fluorescence of one dye-labelled antibody is plotted against that of a second, with the result that a population of cells labelling with one antibody can be further subdivided on the basis of its reactivity with the second antibody.

Immunoassays are another particularly useful form of assay that exploit the specificity, strength and diversity of antibody-antigen reactions or other binding interactions to analyse samples and detect or quantify specific components therein. A wide range of immunoassay techniques are available, such as those described in Wild D. "The Immunoassay Handbook" Nature Publishing Group, 4th Edition, 2013 and subsequent innovations.

Lateral flow assays and more recently non-lateral flow and microfluidics provide a useful set up for biological assays. Such assays can be qualitative, quantitative or semi quantitative. In microfluidic devices, small volumes of liquid are moved through microchannels generated in, for example, a chip or cartridge. A wide range of detection reagents are available including metal nanoparticles, coloured or luminescent materials. Resonance enhanced adsorption (REA) of bioconjugated metal nanoparticles offers rapid processing times and other advantages. These devices have been combined with barcode technologies to identify the patient and the analyte being tested. Computer software and hardware for assessing input data are encompassed by the present disclosure.

A wide range of methods for the detection of antibody to specific antigens are also known. For example, the enzyme-linked immunosorbent assay (ELISA), Western and dot blot assays, and radio-immunoassay (RIA) are routinely used in laboratories. Arrays and high throughput screening methods are also employed.

Qualitative assays providing an intermediate or definitive diagnosis require integrated cutoffs, gates or windows that permit scoring of samples as likely or not to have a condition. Instrument readers and software are often employed to collate data and process it through a diagnostic algorithm or decision tree.

Sepsis is a global healthcare challenge which is reported to have an annual incidence of 56-91 cases per 100 000 people worldwide, with a reported mortality rate of 30% (Jawad, I., et al.. J. Glob. Health 2, 010404 (2012)). Severe infection and sepsis remain significant causes of death and often result in chronic ill-health or disability in those who survive acute episodes. Although sudden, overwhelming infection characteristic of sepsis is comparatively rare amongst otherwise healthy adults, it constitutes an increased risk in immunocompromised individuals, seriously ill patients in intensive care, burns patients and young children. In a proportion of cases, an apparently treatable infection leads to the development of sepsis; a dysregulated, inappropriate response to infection characterised by progressive circulatory collapse leading to renal and respiratory failure, abnormalities in coagulation, profound and unresponsive hypotension and, in about 30% of cases death. Sepsis or severe infections can be rapidly lethal in patients at any age, but it is especially dangerous in children, even more dangerous in neonates, and worst in pre-term infants; each year approximately 15% or 1 million of global neonatal deaths are attributed to neonatal sepsis (severe bacterial infection) (WHO Millennium Development Goal 4). One hurdle in appropriate management and antibiotic treatment for sepsis has been to differentiate between infected and non-infected individuals in the first hours after development of signs or symptoms of sepsis, or more preferably after the early signs of infections that may ultimately lead to sepsis but before the development of more distinctive features such as organ failure. The difficulty in diagnosis for sepsis is described in more detail in the current consensus definition for sepsis (Singer, M. et al. JAMA 315, 801 (2016)).

In some settings, antibiotics may be given to patients showing sepsis- or severe infection-like symptoms in the absence of specific diagnosis, but such treatment may be unnecessary for a majority of patients whose symptoms do not indicate sepsis or severe infection, and such treatment is associated with the risk of adverse drug effects and other complications as well as contributing to the threat of emerging antibiotic resistance, while other patients who do have sepsis or severe infection may not receive timely and appropriate treatment in the absence of specific diagnosis. These problems are most acute in neonatal sepsis, where the combination of non-specific signs and symptoms in these patients together with rapid progression and a high death rate make it very difficult for front-line healthcare workers to make appropriate decisions that can save lives and prevent long-term morbidity in survivors.

Therefore there is a need for an effective diagnostic test that can be used to identify patients with a high likelihood of sepsis or the earlier stages of severe infections that may or may not lead to sepsis, with a strong preference for tests that can give a result in a minimum length of time and without the need for complex laboratory procedures that would limit its use "around the clock" and in resource-poor settings. Preferably, such a test would be applicable to use at the point of care (POC) or near POC.

Blood cultures for infectious organisms (bacteria, yeasts and fungi) are the "gold standard" for the specific diagnosis of sepsis (Mancini, N. et al. Clin. Microbiol. Rev. 23, 235-51 (2010)). However this test requires training of nursing staff in aseptic technique and venipuncture for appropriate collection of large volumes of blood without contamination by skin flora, a laboratory, the procurement of sterile blood culture bottles containing culture fluid, an incubator, and a microscope. Blood culture takes typically 24 to 48 hours to get a result, and requires a trained scientist or sophisticated automated equipment to interpret the results. The decision to initiate or withhold antibiotic therapy must be made much earlier than this to save the life of the individual affected by sepsis.

Approaches to developing POC diagnostics for sepsis have included simple, soluble protein biomarkers that are traditionally considered amenable to POC technologies such as lateral flow immunochromatography. Candidate biomarkers include C-reactive protein (CRP), procalcitonin, IL-10 and other soluble markers of infection and inflammation have been examined (Wagner, T. A. et al.. J. Glob. Health 1, 210-23 (2011)). However none of these soluble biomarkers have demonstrated adequate sensitivity, specificity, or positive predictive value or negative predictive value to be useful as a standalone test to guide clinical decisions in cases of suspected sepsis. For example, CRP is elevated for only a short period during sepsis and is also elevated in many other inflammatory conditions, such as trauma and myocardial infarction.

The measurement of haematological markers, such as elevated or reduced white blood cell count or elevated or reduced neutrophil count, provide some indication of the likelihood of sepsis, but measurement of these markers also requires sophisticated equipment such as automatic analysers or a trained scientist with microscope, and by themselves they are not sufficient for diagnosis.

In accordance with the present disclosure, the most promising biomarkers are likely to be the cell surface biomarkers such as receptors that are induced as part of the innate immune response to a wide range of infections. Ideally, these biomarkers will be elevated early after infection or the onset of sepsis, and will not be affected too quickly by presumptive antibiotic treatment so that patients can still be identified as requiring further support and management even if they have started antibiotics.

One such cell surface biomarker that has been extensively studied and validated as a biomarker of sepsis is the cell surface protein CD64, also known as the high-affinity FC gamma receptor 1 (FCγR1), that binds immunoglobulin (Ig)G with high affinity. While CD64 is constitutively expressed on the surface of monocytes and macrophages, it is expressed at only very low levels on neutrophils in the absence of infection or sepsis, but higher levels of neutrophil expression are rapidly induced during these conditions. CD64 is not expressed on other abundant blood cells such as T-cells but it is expressed on the less numerous dendritic cells. Because of the very low number of circulating dendritic cells relative to neutrophils, they will not be considered further here.

An increasing number of studies on neutrophil CD64 index have been reported (Wagner, T. A. et al. (2011)) highlighting its strong value as a biomarker for sepsis and especially neonatal sepsis. CD64 is a leukocyte surface antigen, the FC gamma receptor 1 (FCγR1), that binds immunoglobulin (Ig)G with high affinity (Masuda, M. & Roos, D. J. Immunol. 151, 7188-95 (1993)). CD64 is part of the three major classes of leukocyte FCγ receptors including FCγRII (CD32) and FCγRIII (CD16) (Masuda, M. & Roos, D. (1993); Hoffmann, J. J. M. L. Clin. Chem. Lab. Med. 47, 903-16 (2009)). The FC receptors, as ligands for immunoglobulin constant regions, play a coordinating role in immunity and mediate functions such as endocytosis, phagocytosis, antibody-dependent cell mediated cytotoxicity (ADCC), and cytokine production (van Vugt, M. J. et al. Blood 94, 808-17 (1999)). CD64 is constitutively expressed on antigen presenting cells (monocytes, macrophages and dendritic cells), to a lesser extent on eosinophils, but only to a very low extent on resting neutrophils (Masuda, M. & Roos, D. (1993); Hoffmann, J. J. M. L. (2009); van Vugt, M. J. et al. Blood 94, 808-17 (1999)). The up regulation of CD64 expression and display on the neutrophil cell surface is considered to be an early step in the innate immune response to bacterial infection (Davis, B. H., Olsen, S. H., Ahmad, E. & Bigelow, N. C. Arch. Pathol. Lab. Med. 130, 654-61 (2006)). The levels of nCD64 remain high from about 3 days after the onset of infection (Aikaterini, et al. CID (2014).

Many studies have looked at neutrophil CD64 expression as a potential biomarker/indicator for detection of sepsis in adults, children and neonates. A previous meta-analysis including 13 studies was published in 2010 (Cid, J., Aguinaco, R., Sánchez, R., García-Pardo, G. & Llorente, A. J. Infect. 60, 313-9 (2010)) and an analysis of 26 studies was published in 2013 (Li, S. et al. Int. J. Infect. Dis. 17, e12-23 (2013)) to evaluate the diagnostic precision of neutrophil CD64 expression for sepsis. The two studies showed a pooled sensitivity of 0.79 (95% confidence interval (CI) 0.70-0.86) and 0.76 (95% CI 0.74-0.78) respectively as well as a pooled specificity of 0.91 (95% CI 0.85-0.95) and 0.85 (95% CI 0.83-0.86) respectively. Given the high numbers of sepsis cases globally, this lower modest sensitivity is consistent with a failure to detect a significant numbers of sepsis positive subjects.

Nevertheless, results suggest that nCD64 ratio could be used as an indicator for differentiating sepsis infected from non-infected patients at a very early stage. However, the need for flow cytometry, sophisticated equipment and experienced and well-trained staff, has to date prevented the widespread adoption of nCD64 ratio even in the developed world, and has almost completely excluded its use in resource-poor settings. Because the nCD64 ratio requires identification of specific surface protein expression (CD64) on a specific subset of white blood cells (neutrophils), it has not been considered as a feasible biomarker for development of a POC sepsis test. Furthermore the sensitivity of nCD64 as a marker for sepsis even in existing flow based tests is not ideal.

Even in the setting of an advanced tertiary care hospital such as The Alfred Hospital, Melbourne, only about 30-40% of strongly suspected sepsis patients in the intensive care unit (ICU) get a laboratory result indicating a positive blood culture. This may be due to early exposure to antibiotics by the GP or emergency room before being admitted into the hospital, ICU. However, the nCD64 value does not change with the exposure of antibiotics (Du, J. et al. PLoS One 9, e102647 (2014); Aikaterini et al. CID (2014)). In addition some patients may have infections with organisms that cannot be cultured using blood culture.

There is thus a need for a rapid diagnostic test for sepsis or severe infection that can help front-line health workers provide timely referral and treatment for the millions of cases of severe bacterial infection in patients admitted in hospitals and ICUs globally, and to exclude the likelihood of such infections in patients with other diseases that may have similar signs and symptoms, such as malaria or viral infections. If it were possible to decrease the time and preferably also the cost to diagnose sepsis, the benefits are clear: increased survival and reduced morbidity in patients of any age, reduced cost and length of hospitalisation, reduced unnecessary exposure to antibiotics and risk of antibiotic resistance, and in the circumstances of neonatal and childhood sepsis, the alleviation of parental worry and infant suffering. Hence, there is a great need for a new rapid, affordable sensitive and specific test for sepsis that can be performed at the point of care. Enhancing the sensitivity of existing platforms for the conduct of neutrophil CD64 assays is also a valuable aspect of the present disclosure.

### SUMMARY

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements.

As used herein the singular forms "a", "an" and "the" include plural aspects unless the context clearly dictates otherwise. Thus, for example, reference to "a composition" includes a single composition, as well as two or more compositions; reference to "an agent" includes one agent, as well as two or more agents; reference to "the disclosure" includes single and multiple aspects of the disclosure and so forth.

The present specification enables an immunoassay suitable for, but not limited to, point of care which is useful for detecting an elevated or altered level of neutrophil CD64 in a biological sample from a subject and assisting in the diagnosis of sepsis or severe infection. The neutrophil CD64 is determined relative to the level of neutrophil elastase (NE), which is as used as a marker of neutrophil number (i.e. a neutrophil number marker, NNM). More particularly, a ratio of neutrophil CD64 to NE is determined, and is used to evaluate neutrophil activation.

The invention accordingly provides an immunoassay for evaluating test sample neutrophil activation for assisting in the diagnosis of sepsis or severe infection in a subject, the assay comprising the steps of:
(i) contacting the sample with an agent that permeabilises or solubilises neutrophils such that total (internal and external) amounts of CD64 are determined;
(ii) simultaneously with (i) or sequentially, contacting the sample with a binding agent that binds specifically to CD64 in the sample and forms CD64-binding agent complex a;
(iii) simultaneously with (i) and/or (ii) or sequentially, contacting the sample with a second binding agent that binds specifically to neutrophil elastase (NE) in the sample and forms NE-binding agent complex b, wherein the amount of NE is proportional to the number of neutrophils in the sample;
(iv) employing the amount of complex a and complex b to determine the relative level of CD64 and of NE in the sample; and
(v) determining a ratio of CD64 and NE (CD64:NE) from the sample, wherein the ratio is internally corrected for the number of neutrophils in the sample determined using the level of NE in the sample and scoring the sample as comprising control or supranormal levels of neutrophil CD64/activation if the CD64:NE ratio exceeds a cut-off level predetermined as above the 95% confidence interval of the line of best fit for the CD64:NE ratio.

In particular, algorithm-based assays are described comprising simple forms suitable for visual determination in point of care lateral flow formats, for example, or more complex forms that require instrumentation and data processing software. Subjects displaying CD64 that is determined to be differentially present are treated for sepsis/severe infection. Antibiotics are currently the first line of defence against sepsis generally broad spectrum antibiotics are administered intravenously.

Reference to sepsis herein includes a severe infection. The term also encompasses a risk of infection and risk of a severe infection. Activated neutrophils display elevated CD64 as an early step of the cascade which will not necessarily lead to sepsis, but will in accordance with the present invention identify severe infections with that risk - and even if not progressing to sepsis, identify severe infections that need to be treated.

The term "differentially present" and the like, is used herein to describe the level of biomarker, and refers to an increase or decrease in the amount of test sample detected CD64 and/or NNM relative to the amount detected in a control subject or control population, and encompasses a higher or lower level of biomarker in a test sample relative to a reference sample.

As described in the Examples below, CD64 and/or NNM (as exemplified by NE) is/are differentially present if its level in a biological sample comprising blood obtained from a test subject is at least 110%, 120%, 130%, 140%, 150%, 160%, 170%, 180%, 190%, 200%, 300%, 400%, 500%, 600%, 700%, 800%, 900% or 1000%, or no more than about 95%, 90%, 80%, 70%, 60%, 50%, 40%, 30%, 20%, 10%, 5%, 4%, 3%, 2%, 1%, 0.5%, 0.1%, 0.01%, 0.001% or 0.0001% of the amount or activity of a corresponding CD64 and/or NNM in a reference sample obtained from a control subject or control population. CD64 and NNM may be differentially present as determined through application of the algorithms described herein to diagnose sepsis or severe infection.

In the assay of the invention, the total amount of CD64 and NE are determined. Accordingly, the assays defined herein comprise contacting the sample with an agent that permeabilises or solubilises neutrophils such that total (i.e. internal and external) amounts of CD64 and NNM are determined.

The term "control sample" includes any sample that can be used to establish reference data and predetermined control CD64 and where appropriate NNM levels from subjects determined to be "healthy" or with a known disease status. The control sample may be a healthy control sample or healthy control samples from a selected reference population of healthy subjects. As will be apparent form the results disclosed herein, samples from control populations exhibit the close relationship between CD64 and NNM (i.e. NE) levels seen in samples from healthy subjects over an appropriate window.

Reference to CD64 and neutrophil elastase (NE) biomarkers includes a modified or homolog form thereof. A modified form includes a derivative, polymorphic variant, truncated form (truncate) and aggregated or multimeric forms or forms having expansion elements (e.g. amino acid expansion elements). Prior art immunoassay methods of determining neutrophil activation and sepsis diagnosis have used an assessment of cell surface CD64 on intact cells. In accordance with the assay of the present invention it has been determined, unexpectedly, that improved diagnosis of sepsis is achieved when total levels of or CD64 and NNM are detected, that is both cell surface bound (extracellular) and internal (intracellular) CD64. Additionally, any soluble CD64 or NE that has been shed from the surface of cells (CD64) or the surface or interior of the cells (NE) into the plasma fraction will also be detected.

An "altered" level means an increase or elevation or a decrease or reduction in the level or ratio of CD64 and/or NE. The determination of the levels of the biomarkers enables establishment of a diagnostic rule based on the levels or ratios relative to controls. In the methods claimed herein, the CD64:NE ratio is used, as defined in the claims The diagnostic rule is based on the application of a statistical procedure. An algorithm uses relationships between biomarkers and disease or healthy status observed in control subjects to infer relationships which are then used to predict the status of patients with unknown status. An algorithm may be employed which provides a visually detectable score or index of probability that a patient is not indicated for sepsis or is indicated for sepsis or severe infection. In some embodiments, an algorithm performs a multivariate or univariate analysis function.

The marker NE was selected to represent the number of neutrophils in a sample, and is expected to show a close correlation with CD64 levels in healthy control patients.

In the assay of the invention, the level of CD64 and the level of NE is detected using binding agents such as without limitation antibody or antigen-binding fragments of antibodies that bind to CD64 or the NE respectively and directly or indirectly provide a detectable signal that can be quantified visually or by instrument. The term "level" or "levels" also encompasses ratios of level/s of biomarkers.

As used herein, "immunoassay" refers to immune assays, typically, but not exclusively sandwich assays, capable of detecting and quantifying a desired biomarker. The immunoassay may be one of a range of immune assay formats known to the skilled addressee.

ECLIA, ELISA and Luminex LabMAP immunoassays are examples of suitable assays to detect levels of the biomarkers. In one example a first binding reagent antibody is attached to a support surface and a second binding reagent/antibody comprising a detectable group binds to the first antibody. Examples of detectable-groups include, for example and without limitation: fluorochromes, enzymes, epitopes for binding a second binding reagent (for example, when the second binding reagent/antibody is a mouse antibody, which is detected by a fluorescently-labeled anti-mouse antibody), for example an antigen or a member of a binding pair, such as biotin. The surface may be a planar surface, such as in the case of a typical grid-type array (for example, but without limitation, 96-welI plates and planar microarrays) or a non-planar surface, as with coated bead array technologies, where each "species" of bead is labelled with, for example, a fluorochrome (such as the Luminex technology described in U. S. Patent Nos. 6,599, 331,6, 592,822 and 6,268, 222), or quantum dot technology (for example, as described in U. S. Patent No. 6,306. 610). Such assays may also be regarded as laboratory information management systems (LIMS):
In the bead-type immunoassays, the Luminex LabMAP system can be utilized. The LabMAP system incorporates polystyrene microspheres that are dyed internally with two spectrally distinct fluorochromes. Using precise ratios of these fluorochromes, an array is created consisting of different microsphere sets with specific spectral addresses. Each microsphere set can possess a different reactant on its surface. Because microsphere sets can be distinguished by their spectral addresses, they can be combined, allowing up to 100 different analytes to be measured simultaneously in a single reaction vessel. A third fluorochrome coupled to a reporter molecule quantifies the biomolecular interaction that has occurred at the microsphere surface. Microspheres are interrogated individually in a rapidly flowing fluid stream as they pass by two separate lasers in the Luminex analyzer. High-speed digital signal processing classifies the microsphere based on its spectral address and quantifies the reaction on the surface in a few seconds *per* sample.

Suitable biological samples include whole blood or blood depleted of certain blood cells or leukocytes such as monocytes and macrophages. In one embodiment, monocytes and macrophages are substantially depleted by an art recognised method such as red cell agglutination or magnetic bead approaches.

The subject contemplated herein is generally a human subject and may also be referred to as a patient, individual or recipient. The human subject may be neonatal or an infant, child, adolescent, teenager, young adult, adult or elderly adult of male or female gender. Control subjects are often selected groups of subjects referred to as a population of subjects/patients. Test samples are generally from subjects suspected of having or being at risk of sepsis however, however samples may also be collected from subjects in individual or general population screening to exclude sepsis or risk of severe infection. Control samples may reflect different subgroups such as neonatal subjects. Any subject group can be usefully employed as a control population including "unhealthy" subjects such as immunocompromised or aged populations.

The binding agent may conveniently be an antibody or an antigen-binding fragment thereof. Other suitable binding agents are known in the art and include antigen binding constructs such as affimers, aptamers, or suitable CD64 or neutrophil marker ligands or parts thereof.

The term "binding agent" and like terms, refers to any compound, composition or molecule capable of specifically or substantially specifically (that is with limited cross-reactivity) binding to an epitope on the biomarker. The "binding agent" generally has a single specificity. Notwithstanding, binding agents having multiple specificities for two or more biomarkers are also contemplated herein. The binding agents (or ligands) are typically antibodies, such as monoclonal antibodies, or derivatives or analogs thereof, but also include, without limitation: Fv fragments; single chain Fv (scFv) fragments; Fab' fragments; F(ab')2 fragments; humanized antibodies and antibody fragments; camelized antibodies and antibody fragments, and multivalent versions of the foregoing. Multivalent binding reagents also may be used, as appropriate, including without limitation: monospecific or bispecific antibodies; such as disulfide stabilized Fv fragments, scFv tandems [(scFv) 2 fragments], diabodies, tribodies or tetrabodies, which typically are covalently linked or otherwise stabilized (i.e. leucine zipper or helix stabilized) scFv fragments. "Binding agents" also include aptamers, as are described in the art.

Methods of making antigen-specific binding agents, including antibodies and their derivatives and analogs and aptamers, are well-known in the art. Polyclonal antibodies can be generated by immunization of an animal. Monoclonal antibodies can be prepared according to standard (hybridoma) methodology. Antibody derivatives and analogs, including humanized antibodies can be prepared recombinantly by isolating a DNA fragment from DNA encoding a monoclonal antibody and subcloning the appropriate V regions into an appropriate expression vector according to standard methods. Phage display and aptamer technology is described in the literature and permit in vitro clonal amplification of antigen-specific binding reagents with very affinity low cross-reactivity. Phage display reagents and systems are available commercially, and include the Recombinant Phage Antibody System (RPAS), commercially available from Amersham Pharmacia Biotech, Inc. of Piscataway, New Jersey and the pSKAN Phagemid Display System, commercially available from MoBiTec, LLC of Marco Island, Florida. Aptamer technology is described for example and without limitation in US Patent Nos. 5,270,163; 5,475,096; 5,840,867 and 6,544,776.

The present methods employ the observed non-linear positive correlation between CD64 and NNM as described for the diagnosis of sepsis or severe infection.

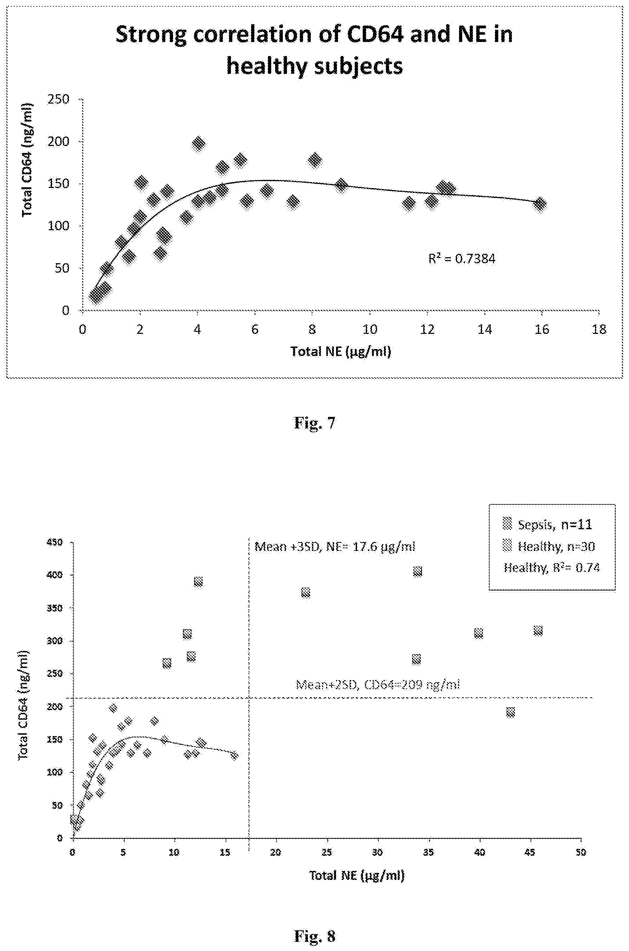

In further experiments, to improve the ability of the assays to diagnose sepsis or severe infection a close correlation was determined between NE and CD64 levels in healthy patients (Figure 7). This provides a "gate" of healthy levels for these two markers, with sepsis patients falling outside this healthy "gate". The observation that CD64 levels per cell (or unit of NE) are higher at lower values of NE and reach a plateau value at NE of around 2.5-5.0 µg/ml can also be controlled for in a decision tree.

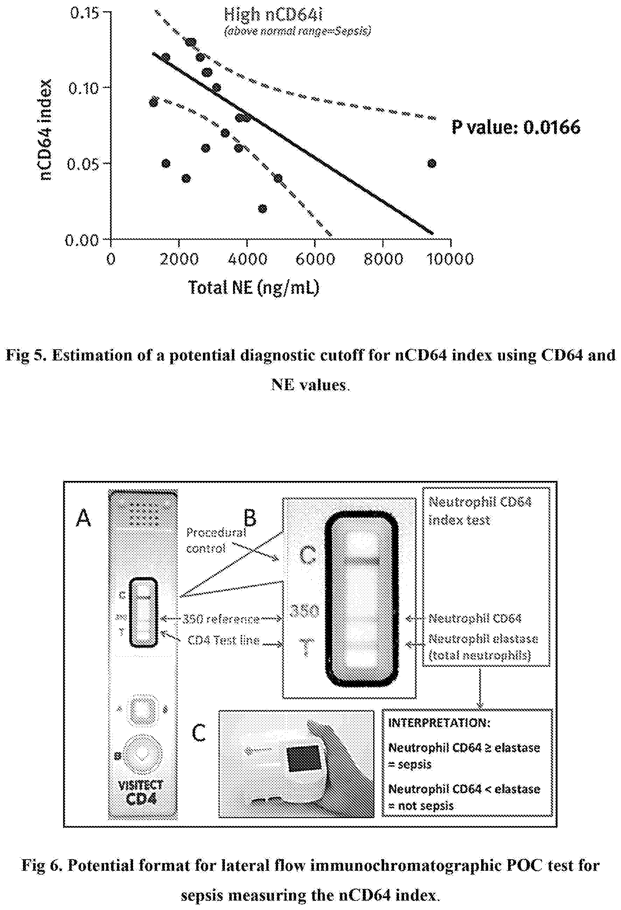

Accordingly, while one option is to determine the arithmetic relationship of nCD64i vs NE as anticipated in Figure 5, further gating approaches have been developed in light of the above observation illustrated in Figure 7.

The skilled person will be able to design variant gating strategies aiming at a similar result.

The "complex" cutoff (adjusted for neutrophil number by using the NE/NNM and an algorithm) are suited to an automated reader instrument such as the Axxin AX-2x.

Embodiments of the subject assays are described by comparing the ratio of CD64 and NE in test samples relative to the ratio found in healthy control samples. Thus, elevated levels compared to controls (i.e. above the 95% confidence interval of the line of best fit for the CD64:NE ratio) are indicative of activated neutrophils and sepsis. It is apparent to the skilled person that non-sepsis subjects may be identified in the test samples by seeking alignment with control "healthy" levels.

A positive score for sepsis or severe infection or risk of same in accordance with the present disclosure and invention permits prompt administration of antibiotics where appropriate. The present assay may therefore be used in the treatment and/or prophylaxis of sepsis or severe infection.

In one embodiment the sample is a whole blood cell sample. In another embodiment the sample is or has been depleted of one or more leucocytes such as monocytes or macrophages.

The assay claimed herein comprises diagnosing the subject as having supranormal amounts of neutrophil CD64/activation if the CD64/NE ratio exceeds a cutoff level predetermined as a function of the amount of the NE in the test sample (internally corrected for neutrophil number). The ratio of CD64 to NE in the sample is internally corrected for the number of neutrophils in the sample determined using the level of NE in the sample.

In one embodiment the assay is a point-of-care assay.

In one embodiment the assay is an enzyme-linked immunosorbent (ELISA)-type, flow cytometry, bead array, lateral flow, cartridge, microfluidic or immunochromatographic based method or the like.

In one embodiment the binding agent is an antibody or an antigen-binding fragment or derivative thereof, an antigen-binding construct such as an affimer, aptamer or a ligand or binding part thereof.

In one embodiment the binding agent is immobilised on a support.

In one embodiment, in step (ii), the sample is contacted with the binding agents of step (ii) and (iii) by applying the sample to a sample portion of an immunoassay device wherein the device sample portion is operably connected to spaced capture portions of the device and whereby the components of the sample flow from the device sample portion to and through the device capture portions, and wherein one capture portion comprises the binding agent which specifically binds to CD64 in the sample such that the CD64 is captured by the binding agent to form a binding agent-CD64 complex in the capture portion, and wherein a second capture portion comprises the binding agent which specifically binds to NE in the sample such that the NE captured by the binding agent to form a binding agent-neutrophil number marker complex in the capture portion.

In one embodiment the amount of CD64 complex and the amount of NE binding agent complex is detected using a binding agent such as an antibody or antigen-binding fragment, ligand, aptamer or affimer that binds to CD64 or NE respectively and directly or indirectly provides a detectable signal that can be quantified visually or by instrument.

In one embodiment the instrument comprising software is used to input data based on the observed amounts/levels of CD64 and NE and optionally manage/process the data relative to a database comprising data from control subjects/populations following the diagnostic rule as claimed.

In one embodiment the binding agent is conjugated to a detectable marker or microparticles comprising a detectable marker, that provide a detectable signal.

In one embodiment the capture portion is a test line.

In one embodiment the sepsis is neonatal sepsis.

The assay described herein is suitable for rapid point of care assays and devices such as lateral flow devices where for example, the simple algorithms described herein can be deployed through the medium of visually or instrument detectable test lines representing levels of biomarkers.

The assay described herein permits integration into existing or newly developed pathology architecture or platform systems. For example, the method described herein allows a user to determine the status of a subject with respect to a pathophysiological condition associated with neutrophil activation such as sepsis or severe infection, the assay including:
(a) receiving data in the form of the ratio of CD64: NE in a test sample from the user via a communications network;
(b) processing the subject data via an algorithm which provides a score or disease index value by comparing the ratio of CD64:NE to those from predetermined control levels.

In some embodiments, an indication of the status of the subject to the user is transferred via a communications network. It will also be appreciated that in one example, the end stations can be hand-held devices, such as PDAs, mobile phones, or the like, which are capable of transferring the subject data to the base station via a communications network such as the Internet, and receiving the reports. When a server is used, it is generally a client server or more particularly a simple object application protocol (SOAP).

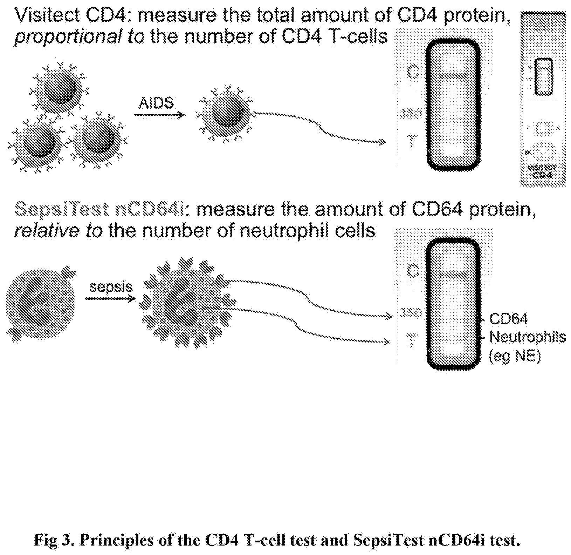

Example 1 and Figures 3 to 6 describe a cutoff for sepsis based upon measuring relative levels of CD64 and NE. The further examples and Figures illustrate a simplified analytical method based upon the original findings, in which the close correlation between NE and CD64 levels in healthy patients (Figure 7) provides a "gate" of healthy levels for these two markers, with sepsis patients falling outside this healthy "gate" - but still with the observation that CD64 levels per cell (or unit of NE) are higher at lower values of NE and reach a plateau value at NE of around 2.5-5.0 µg/ml.

Aspects of the present disclosure provide numerical values in various ranges. Slight variations above and below the stated ranges can be used to achieve substantially the same results as values within the ranges. Also, these ranges are intended as a continuous range including every value between the minimum and maximum values. In addition, the present disclosure extends to ratios of CD64 and NE levels providing a numerical value associated with a state of neutrophil activation, sepsis or severe disease.

In one embodiment, it may be clarified that markers for monocyte/macrophage cells, such as CD163, are not required as controls.

The above summary is not and should not be seen in any way as an exhaustive recitation of all embodiments of the present disclosure.

### BRIEF DESCRIPTION OF THE FIGURES

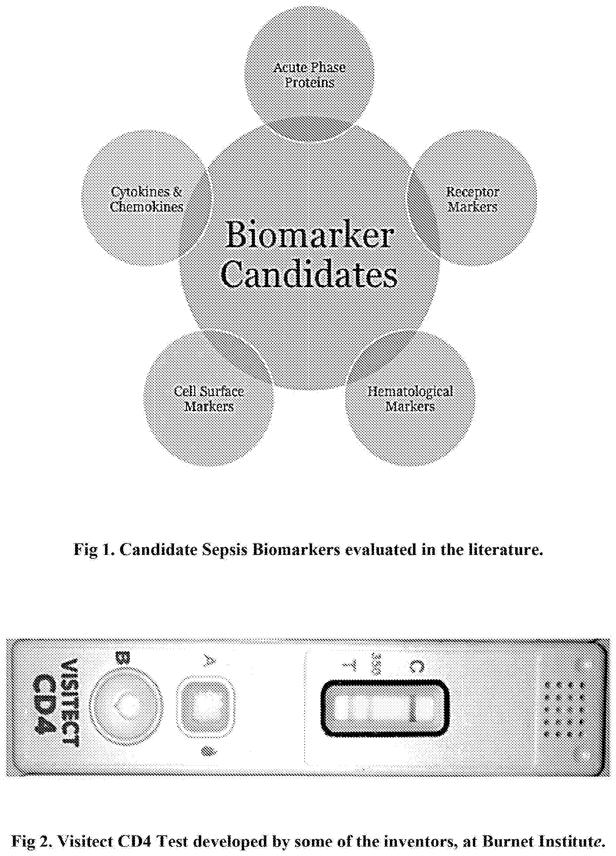

**Figure 1** illustrates the large number of candidate Sepsis Biomarkers evaluated in the literature.
**Figure 2** illustrates that Visitect CD4 Test developed by some of the inventors, at Burnet Institute (produced under license by Omega Diagnostics, UK). In the CD4 T-cell test, monocytes and macrophages are depleted from the whole blood sample (30 µl) by interaction with RosetteSep reagent (StemCell Technologies, Vancouver, Canada), causing their agglutination with red blood cells in the sample pad (well "A"). The remaining blood fraction containing plasma and white blood cells apart from monocytes/macrophages flows towards the test zone and interacts with detergent (Triton X-100), lysing the white blood cells and solubilising cell-associated full-length CD4 on T-cells in the sample. The sample then flows past the test line, being a binding reagent (monoclonal antibody) directed against the cytoplasmic domain of CD4, found only in cell-associated CD4 and not in the fraction of soluble CD4 in plasma. The captured CD4 is then detected with colloidal gold complexed with a second monoclonal antibody, directed against the extracellular domain of CD4, initiated by addition of buffer to the gold conjugate pad (well "B"). The intensity of the test line is therefore representative of the total amount of T-cell associated CD4 in the sample, and by inference the number of CD4 T-cells because the amount of CD4 molecules per cell is substantially constant. That is, the amount of CD4 is directly proportional to the number of CD4 T-cells. The test signal is then compared visually or by instrument reader versus one or more reference lines representing clinically relevant levels of CD4 T-cells, in the figure the example of 350 T-cells per µl which is recommended by WHO for prioritisation of antiretroviral therapy in HIV infection.
**Figure 3** illustrates the principles of the CD4 T-cell test and SepsiTest nCD64i test. In the condition of HIV infection progressing towards acquired immunodeficiency syndrome (AIDS), the number of CD4 T-cells may decline, while the amount of CD4 on each remaining cell remains constant. The amount of CD4 is therefore proportional to the number of CD4 T-cells present in the same. In the condition of invasive infection or sepsis, the number of neutrophils may increase, decrease or stay the same, but the level of expression of CD64 (shown as blue molecules on the surface of the cells) is substantially increased. The number of neutrophils can be measured in the same way as the number of T-cells using an antigen that is associated with the neutrophils, such as but not limited to neutrophil elastase. The amount of CD64 can be measured in the same way as CD4, and either cell-associated or both cell-associated and soluble CD64 may be measured using methods well known in the art including methods for exclusively cell-associated transmembrane proteins (US Patent 8,409,818 and other territories). However in the case of CD64 the amount per cell will be either low in healthy individuals, or elevated in the case of severe infection or sepsis, and this difference will be detected as an increase in the ratio between CD64 and the neutrophil-specific protein (neutrophil elastase in this example).

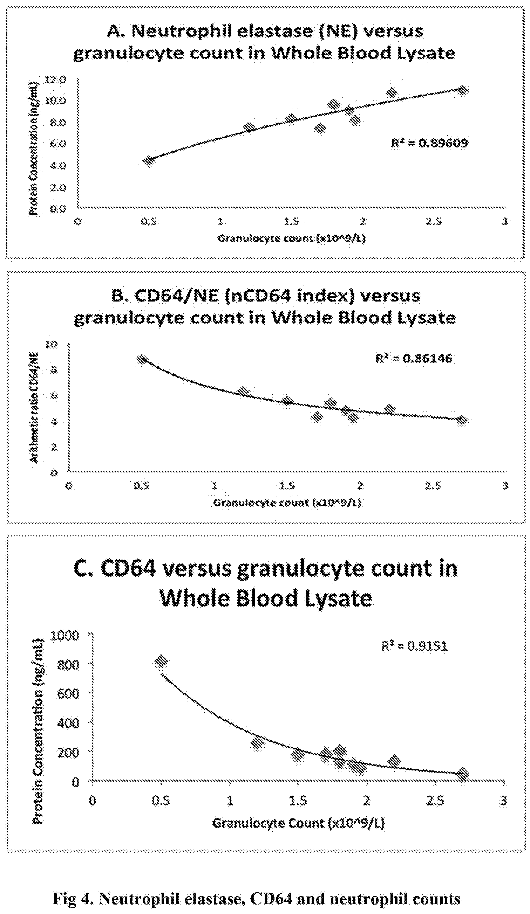

**Figure 4** is a graphical representation of neutrophil elastase, CD64 and neutrophil counts among healthy individuals. 4A shows the good correlation (R2= 0.89609) between ELISA Neutrophil Elastase (Neutrophil specific marker) and Neutrophil (Granulocyte) count. 4B shows good correlation (R2= 0.86146) between CD64/NE ratio (nCD64 index) and Granulocyte count, but a surprising higher level of CD64 index at lower neutrophil counts. 4C shows good correlation (R2= 0.9151) for CD64 versus granulocyte count in Whole Blood Lysate, with the unexpected observation that CD64 amounts are higher in samples with lower neutrophil (granulocyte) count, leading to the observed variable relationship seen in Figure 4B.
**Figure 5** is a graphical representation showing estimation of a potential diagnostic cutoff for nCD64 index using CD64 and NE values. The total amount of NE and CD64 was measured in monocyte-depleted whole blood from healthy adult volunteers, and the nCD64 index ratio calculated. The results show a highly significant correlation (P= .0166) and again show the variable relationship with higher nCD64i at lower neutrophil counts. The dashed red lines show the lower and upper 95% confidence intervals for the relationship, and it is predicted that samples with nCD64i falling above the upper 95% confidence interval for the measured NE concentration would have a strong suspicion of sepsis.
**Figure 6** illustrates a format for lateral flow immunochromatographic POC test for sepsis measuring the neutrophil CD64 index. Visitect CD4 test (A), expanded view and schematic of proposed nCD64 index test (B, red boxes), and Axxin AX-2 reader (C). For visual interpretation an intensity for the CD64 line greater than the NE line might be considered to be diagnostic, however with the use of a reader such as the Axxin AX-2 reader (Fig. 6C) the cutoff would be adjusted according to the total amount of neutrophils (NE quantity), and the appropriate cutoff calculated for the instant patient sample.
**Figure 7** shows results of ELISA for NE and CD64 in whole blood for healthy subjects. NE and CD64 were assessed via ELISA in whole blood for healthy subjects (n=30), and shows that in healthy subjects, the total amount of CD64 in whole blood is closely correlated with the total amount of NE in whole blood (R2= 0.74).
**Figure 8** shows the results of ELISA for NE and CD64 in whole blood for sepsis patients. NE and CD64 were assessed via ELISA in whole blood for sepsis patients (red markers, n=11) together with the healthy subjects as shown in Figure 7 (blue markers).

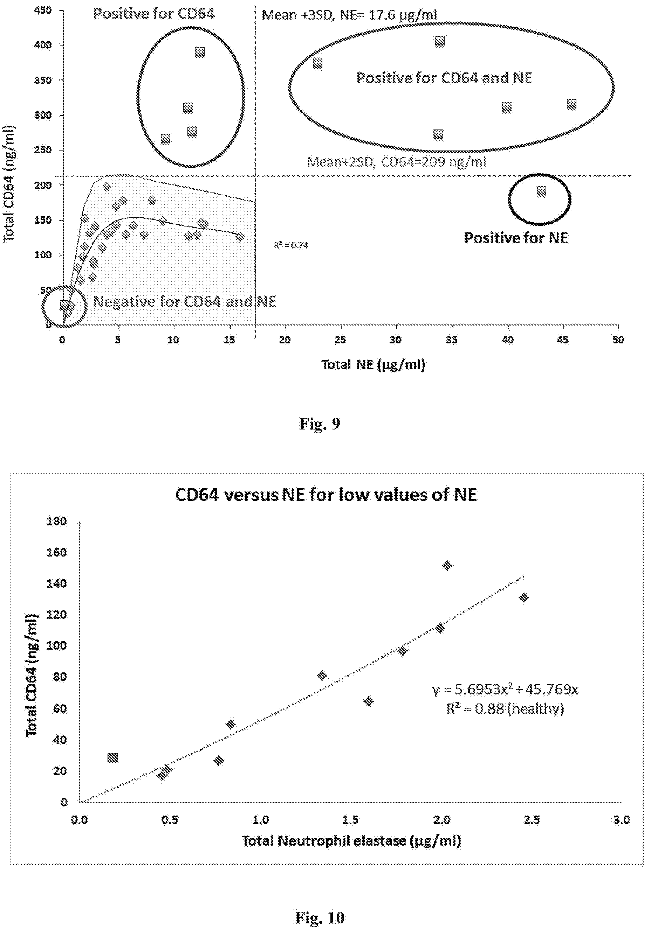

**Figure 9** shows the same results as Figures 7 and 8 but highlights the four different classifications of sepsis patient samples according to the cutoffs based on the mean plus 2 SD of CD64 and mean plus 3 SD of NE. By these criteria, one patient is positive only for NE, 5 patients are positive for both NE and CD64, 4 patients are positive for CD64 only, while one patient is negative for both NE and CD64, with very low values for both markers, and suggesting that the patient may have low levels of neutrophils.
**Figure 10** shows the analysis of CD64 versus NE for low values of NE. The X axis shows total CD64 as ng/mL and the X axis shows the total NE as µg/mL.

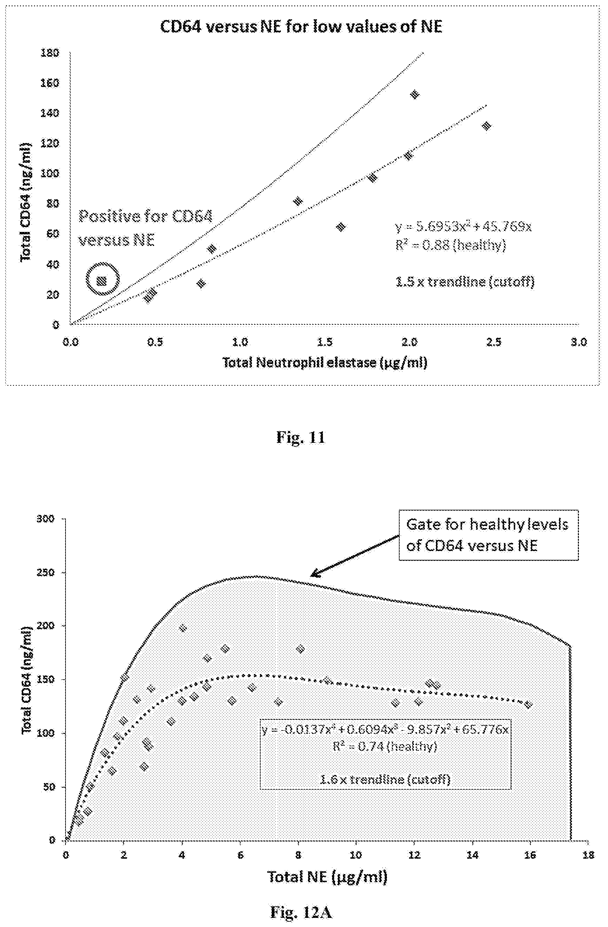

**Figure 11** shows the analysis of CD64 versus NE for low values of NE. The X axis shows total CD64 as ng/mL and the X axis shows the total NE as µg/mL.

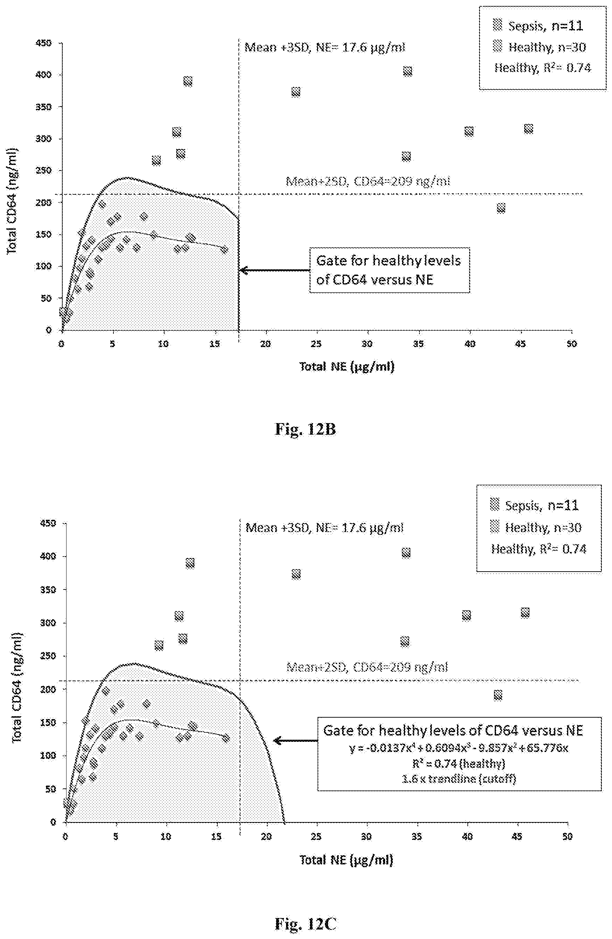

**Figure 12** shows the gate for healthy levels of CD64 versus NE. Panel A, B and C.

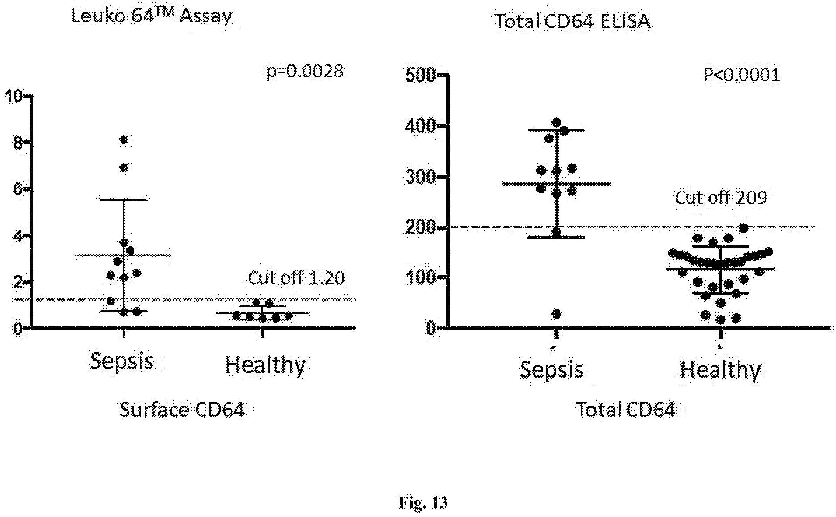

**Figure 13** illustrates the results of a comparison of Leuko64 and the subject assay (in ELASA format) to identify sepsis patients. Leuko64 showed highly significantly higher values for sepsis patients than for healthy controls, but using the manufacturer recommended cutoff of 1.2, only 8/11 sepsis patients were identified with this test, consistent with many reports in the literature that show around 70-80% sensitivity of this method. Similarly, detection of total whole blood CD64 alone by ELISA showed highly significantly higher values for sepsis patients than for healthy controls, but using the cutoff of mean plus 2 SD (209 ng/ml), only 9/11 sepsis patients were identified with this test.

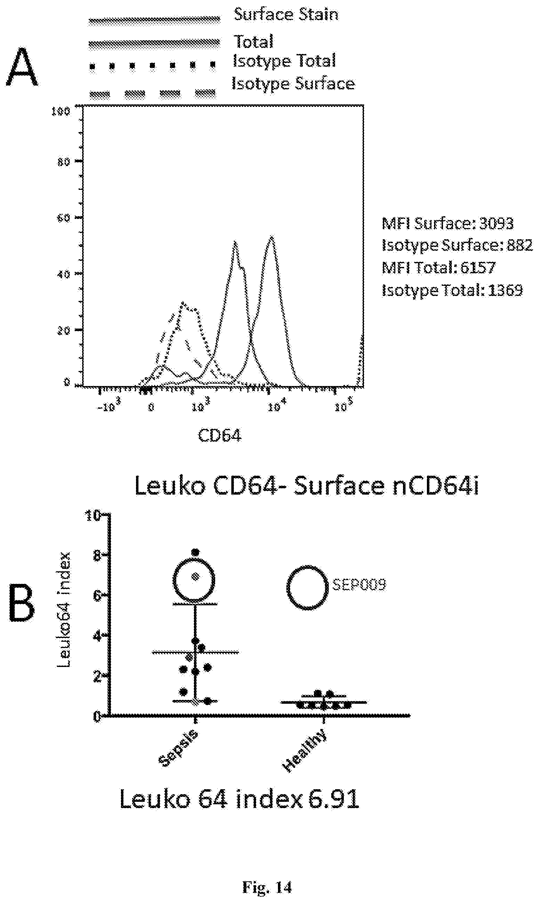

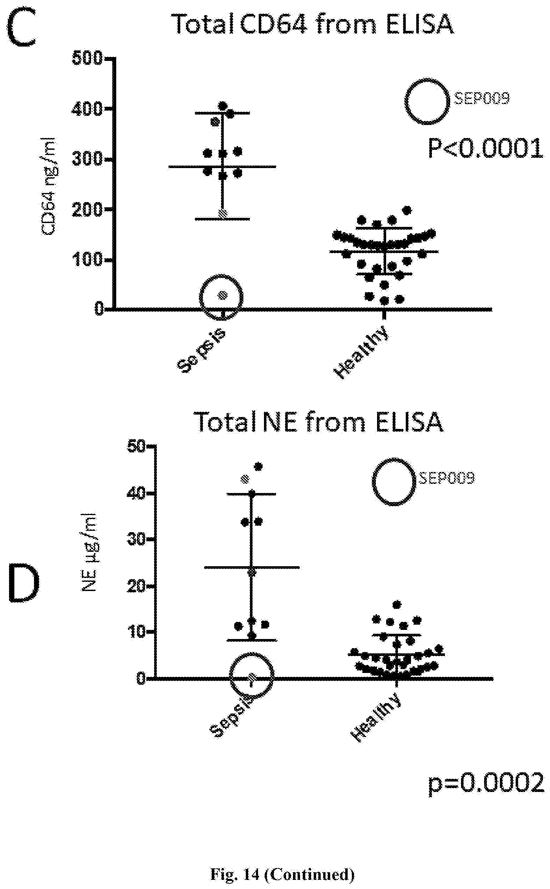

**Figure 14** shows the results of this analysis (Figure 13) for patient SEP009, the patient with the lowest levels of NE in Figure 13. In panel A, it is shown that the neutrophils from this patient have high levels of surface expression of CD64 (red line) consistent with very high relative CD64 expression in Leuko64 (panel B), but even higher levels of CD64 per cell when intracellular CD64 is detected as well (blue line). However the low level of NE (panel D) and presumed low neutrophil counts for this patient result in a total CD64 result that is below the cutoff based on CD64 alone (panel C), but detectable using the algorithm approach illustrated in Figures 8-12 and described herein. This result also suggests that for patients with low levels of NE, the Leuko64 approach or other methods may be of additional utility given the very high Leuko64 result for this patient.

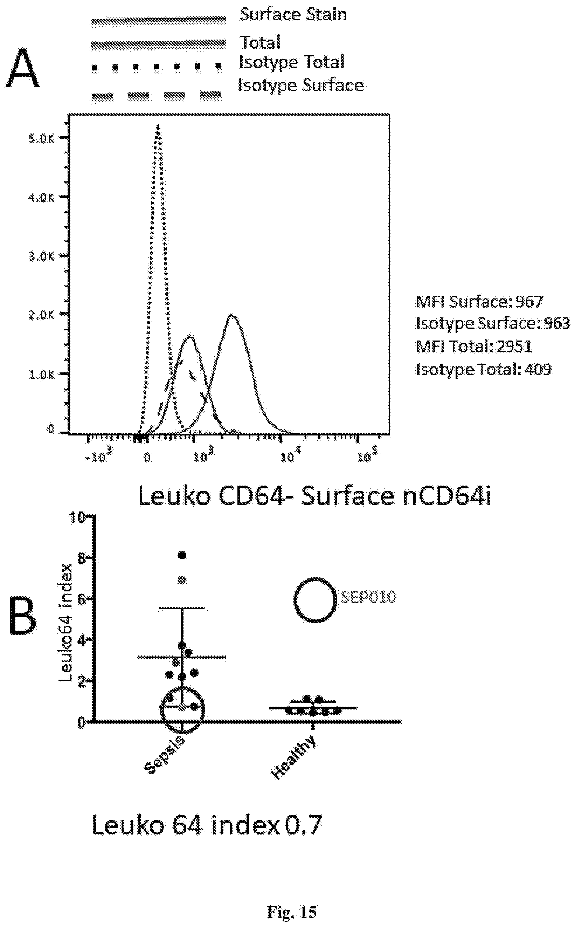

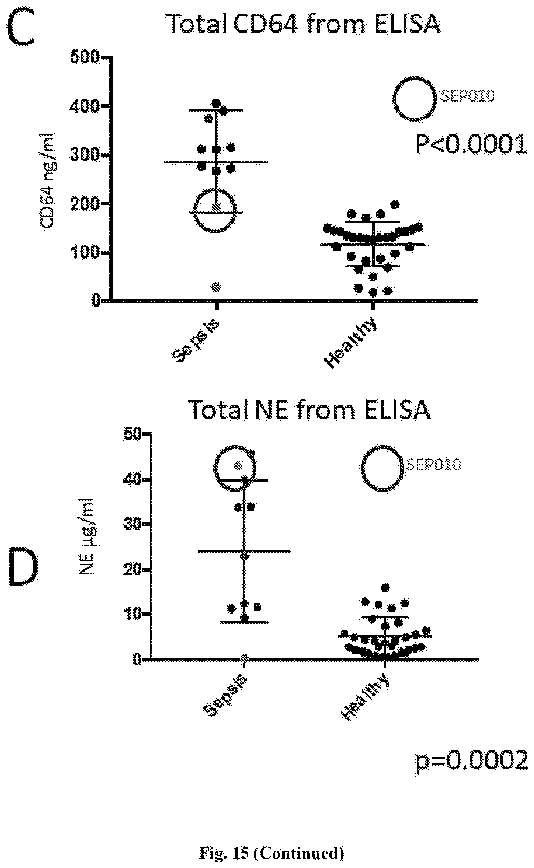

**Figure 15** shows the same analysis for patient SEP010, the patient with elevated levels of CD64 but just below the cutoff in Figure 9 derived from the mean plus 2 SD of CD64 from healthy subjects. In this patient, there was minimal surface staining of CD64 (panel A, comparing red line and isotype control for surface staining) consistent with low relative CD64 expression in Leuko64 (panel B), but there were high levels of CD64 detected when intracellular CD64 was stained as well (panel A blue line). Consistent with this observation, patient SEP010 had modest levels of total CD64 by ELISA, although still just below the cutoff for total CD64 based on mean plus 2SD (panel C), whereas the Leuko64 result of 0.7 was well below the manufacturer cutoff of 1.2 for that test (panel B). However, when NE is examined the patient SEP010 is seen to have very highly elevated levels of NE, providing unequivocal diagnosis of sepsis using the cutoff derived from the mean plus 3SD of NE, and also using the cutoff derived from the trendline equation.

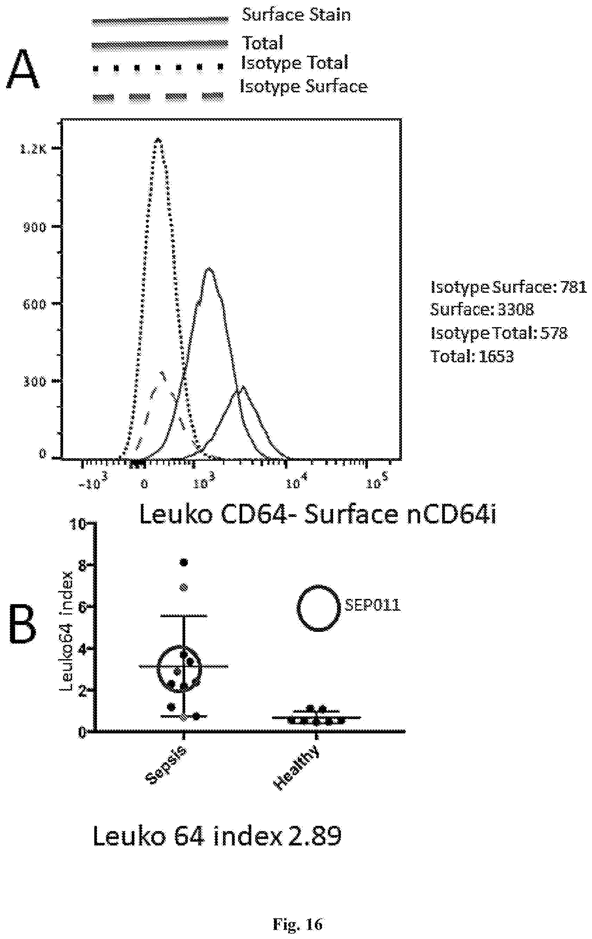

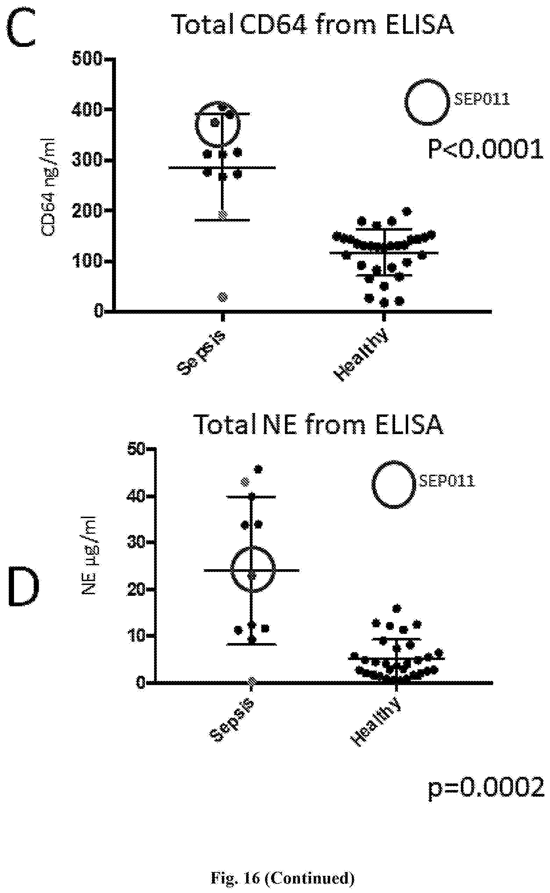

**Figure 16** shows the same analysis for patient SEP011, a patient with elevated levels of both CD64 and NE. In this patient, there was significant surface staining of both intracellular and surface CD64 (panel A, comparing red line and isotype control for surface staining, blue line and isotype control for total staining) consistent with high relative CD64 expression in Leuko64 (panel B), well above the manufacturer cutoff. Patient SEP011 had highly elevated levels of total CD64 by ELISA (panel C), and also highly elevated levels of NE (panel D), providing unequivocal diagnosis of sepsis using the cutoff derived from the mean plus 3SD of NE, AND the cutoff derived from the mean plus 2SD of CD64, AND also using the cutoff derived from the trendline equation.

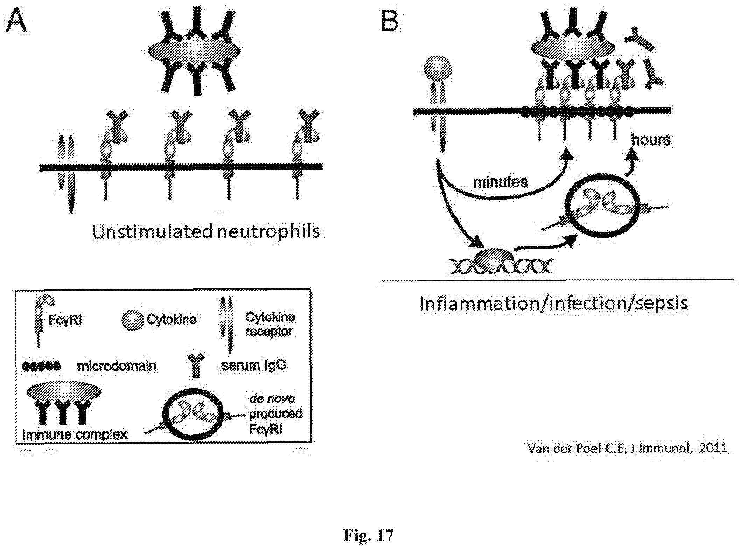

**Figure 17** is an illustration, modified from van der Poel (2011), showing how in healthy individuals, low levels of CD64 (FcγR1) are expressed on the cell surface, and these are saturated by monomeric IgG which does not result in cell signaling or antigen complex internalization (Figure 17A). Upon initial stimulation of the neutrophil by interferon gamma or other stimuli associated with bacterial infection or sepsis, additional CD64 is synthesized and translocated to the surface of the neutrophil and associated with membrane microdomains, together allowing for binding of multimeric Ig in immune complexes which leads to cell signaling (Figure 17B).

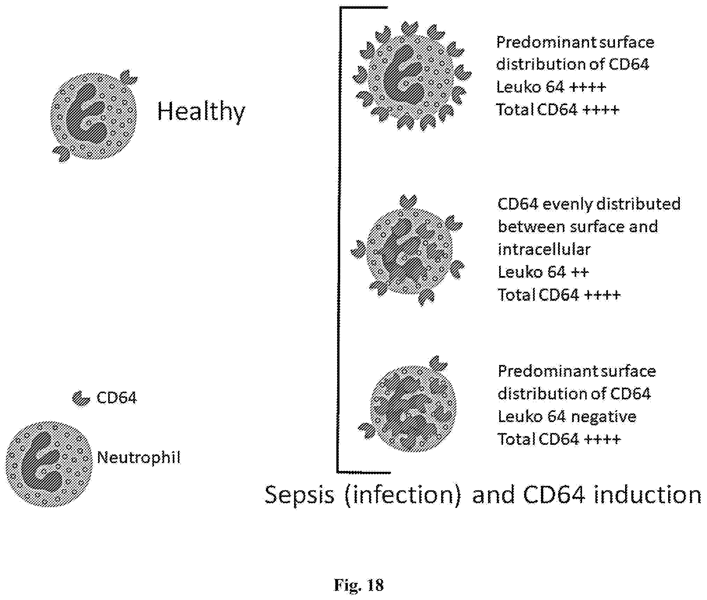

**Figure 18** illustrates activated neutrophils having different levels of surface CD64, even though they have the same elevated level of total CD64. On the left, a "healthy" neutrophil is shown schematically with low levels of CD64, expressed on the surface. These cells may also contain small amounts of pre-formed, intracellular CD64. In sepsis, "activated" neutrophils are shown on the right, each with equally large amounts of CD64, significantly increased compared to healthy neutrophils, but with distribution on the surface (similar to the patient sample SEP09, Figure 14), or evenly distributed between the surface and intracellular (similar to the patient sample SEP011, Figure 16), or predominantly distributed to the intracellular compartment (similar to patient sample SEP010, Figure 15). As such, any method that allows the detection of both cell surface and intracellular CD64 is likely to allow improved diagnosis of sepsis.

### DISCUSSION OF EMBODIMENTS

Unless defined otherwise, all technical and scientific terms used herein have the same meanings as commonly understood by one of ordinary skill in the art to which this disclosure belongs. Any materials and methods similar or equivalent to those described herein can be used to practice or test the present disclosure. Practitioners are particularly directed to Wild D. "The Immunoassay Handbook" Nature Publishing Group, 4th Edition, 2013 and Ausubel et al., Current Protocols in Molecular Biology, Supplement 47, John Wiley & Sons, New York, 1999; Colowick and Kaplan, eds., Methods In Enzymology, Academic Press, Inc.; Weir and Blackwell, eds., Handbook of Experimental Immunology, Vols. I-IV, Blackwell Scientific Publications, 1986; for definitions and terms of the art and other methods known to the person skilled in the art. Immunoassays can be done in any convenient format known in the art.

Antibodies are often used in immunoassays because of the ability to produce them in large quantities and the homogeneity of the product . The preparation of hybridoma cell lines for monoclonal antibody production is derived by fusing an immortal cell line and lymphocytes sensitized against the antigen of interest or can be done by techniques which are well known to those who are skilled in the art. (See, for example, Douillard and Hoffman, Basic Facts about Hybridomas, in Compendium of Immunology Vol. II, ed. by Schwartz, 1981; Kohler and Milstein, Nature 256: 495-499, 1975; European Journal of Immunology 6: 511-519, 1976 or more recent references Sambrook, Molecular Cloning: A Laboratory Manual, 3rd Edition, CSHLP, CSH, NY, 2001). The DNA that encodes antibodies can be manipulated in vitro and introduced back into lymphoid cell lines, thus allowing the production of genetically-engineered antibodies. In the last decade, the use of transient mammalian expression systems for the production of native complex proteins has increased (see S. Geisse, B. Voedisch Methods Mol. Biol., 899 (2012), pp. 203-219) boosted by the publication of efficient transfection protocols and the availability of suspension cell lines growing at high density. For transient expression, mainly the HEK-293 and CHO-K1 cell lines have been applied. Both cell lines can be adapted to suspension culture, and sub clones are available which grow in chemically defined medium at high cell densities. With its ease of transfection, high expression yield and native human glycosylation, the human embryonic kidney 293 cell line may be used.

Antibodies (and their smaller formats such as scFv and Fab fragments can be produced in any cell type known in the art. Cell-free protein synthesis, also termed in vitro translation, facilitates the production of a given target protein by utilizing the translational machinery without using the living cell. Cell-free systems have been successfully used for the high-throughput production of protein libraries as well as for the high-yield synthesis of selected target proteins. In particular, the use of linear DNA templates contributes to the ease and speed of cell-free translation systems, since no time-consuming cloning steps are required prior to protein production. Antibodies of a given target protein takes approximately one to two days, whereas cell-based expression, including the cloning procedure and cell-transformation, may take up to two weeks. Phage display technology is the most commonly used technique for the in vitro selection and evolution of antibody fragments.

Alternatives to antibodies as specific binding agent are described in the literature and are recognised in the field for their potential to improve *inter alia,* assay reproducibility and stability. A review of antibody alternatives is provided by McLeod et al The Scientist February 2016, and includes aptamers and affimers. Any such binding agent may be employed in the present assays and kits without undue experimentation.

The presence of complexes may be evaluated using ELISA-type procedures. A wide range of immunoassay techniques are available as can be seen in U.S. Pat. Nos. 4,016,043, 4,424,279 and 4,018,653. These include both single-site and two-site or "sandwich" assays of the non-competitive types, as well as in the traditional competitive binding assays. The skilled person will appreciate that the selection and implementation of a known label system involves only routine experimentation.

Sandwich assays are among the most useful and commonly used assays. A number of variations of the sandwich assay technique exist, and all are intended to be encompassed by the present invention. Briefly, in a typical forward assay, an binding agent is immobilized on a solid or semi-solid substrate and the sample to be tested brought into contact with the bound molecule. After a suitable period of incubation, for a period of time sufficient to allow formation of an binding agent-antigen complex, a second binding agent specific to the antigen, labelled with a reporter molecule capable of producing a detectable signal is then added and incubated, allowing time sufficient for the formation of another complex of binding agent-antigen-labelled binding agent. Any unreacted material is washed away, and the presence of the marker is determined by observation of a signal produced by the detectable marker (reporter molecule). The results may be qualitative or quantitative, by simple observation of the visible signal, or may be quantitated by comparing with a control sample containing known amounts of marker. Variations on the forward assay include a simultaneous assay, in which both sample and labelled binding agent are added simultaneously to the bound binding agent. These techniques are well known to those skilled in the art, including any minor variations as will be readily apparent. In accordance with the present invention, the sample is generally a biological sample comprising biological fluid, and is most conveniently a whole blood sample such as capillary or venous blood that may be treated with an anticoagulant.

In a typical forward sandwich assay, a first binding agent having specificity for a marker is either covalently or passively bound to a solid or semi-solid support. The support is typically glass or a polymer, the most commonly used polymers being nitrocellulose, cellulose, polyacrylamide, nylon, polystyrene, polyvinyl chloride, polypropylene or mixture or derivatives of these. The solid supports may be in the form of tubes, beads, discs or microplates, or any other surface suitable for conducting an immunoassay. The binding processes are well-known in the art and generally consist of cross-linking covalently binding or physically adsorbing the polymer-binding agent complex to the solid surface which is then washed in preparation for the test sample. An aliquot of the sample to be tested is then added to the solid phase complex and incubated for a period of time sufficient (e.g. 2-40 minutes or overnight if more convenient) and under suitable conditions (e.g. from room temperature to about 37°C. including 25°C.) to allow binding of any subunit present in the binding agent. Following the incubation period, the binding agent subunit solid phase is washed and incubated with a second binding agent specific for a portion of the antigen. The second binding agent is linked to a detectable marker which is used to indicate the binding of the second binding agent to the antigen.

An alternative method involves immobilizing the target molecules in the biological sample and then exposing the immobilized target to specific binding agent which may or may not be labelled with a detectable marker. Depending on the amount of target and the strength of the signal from the detectable marker, a bound target may be detectable by direct labelling with the binding agent. Alternatively, a second labelled binding agent, specific to the first binding agent is exposed to the target-first binding agent complex to form a target-first binding agent-second binding agent tertiary complex. The complex is detected by the signal emitted by the reporter molecule. A significant improvement of the bead-based methods involves tagging each bead with a unique identifier tag, such as an oligonucleotide or electrophoretic tag, so as to facilitate identification of the amino acid sequence of each library member. These improved bead-based methods are described in International Publication No. WO 93/06121.

In other embodiments, the method is a liquid phase method. In one example of a liquid phase immunoassay (see for example U.S. Pat. No. 6,632,603) the sample is contacted with an agent capable of binding a marker and a detector agent comprising a visually detectable agent such as colloidal gold or silver labelled. The test sample is applied by flowing onto a defined zone of an insoluble porous support film having a pore size impassable to a complex formed between the marker and its target, and if present, with a binding substance and a detector substance, but passable to the binding substance and detector substance while remaining uncomplexed in the absence of the desired target. If the target is present in the test specimen, the detector substance binds with the target and the binding substance to form a visually inspectable complex on the surface of the porous support film. After application of the test sample to the porous support, the surface of the porous support is visually inspected for colour to determine the presence and quantity or the absence of the marker being assayed.

In another assay, magnetic antibodies that bind to markers are used to tag markers and a high Tc superconducting quantum interference device is used to measure the amount of target protein. A liposome immunomigration, liquid-phase competition strip immunoassay is, for example, described in Glorio-Paulet et al J Agric Food Chem 48 (5):1678-1682, 2000.

General formats and protocols for the conduct of various formats of ELISA are disclosed in the art and are known to those of skill in the field of diagnostics. For example, reference may be made to Chapter 11 of Ausubel (Ed) Current Protocols in Molecular Biology, 5th Edition, John Wiley & Sons, Inc, NY, 2002. Rundsträm, G et al. describe lateral Flow immunoassay using Europium (III) Chelate Microparticles and Time-Resolved Fluorescence for eosinophils and neutrophils in Whole Blood. Clinical Chemistry 53, 342-348 (2007).

Various methods are available for depleting monocytes or red blood cells from blood, if required. In some embodiments, monocytes in the sample are depleted by contacting the sample with anti-CD14 or other relevant antibodies bound to a solid or semi-solid support. In another embodiment, red blood cells are depleted in the sample by contacting the sample with anti-glycophorin A antibodies bound to a solid or semi-solid support. A chromatographic device may be used comprising material which has a pore size which allows or facilitates capillary flow of the components of the method. The device may comprises portions comprising material of different pore size, or non-porous material, the material being contiguous with the first material and designed to receive a sample or receive or store components of the method. The portions of the chromatographic device may be separate, contiguous or overlapping or designed to come together in use.

The sample pad may be chromatographically connected to a test portion of the device, the test portion comprising a binding agent such as an antibody or an antibody binding fragment thereof, an antigen-binding constructs such as an affimers or ligands or parts thereof. For example, the subject is a mammal and the test portion comprises an antibody or affimer which, under the appropriate conditions, recognizes and binds CD64. For example, the subject is a mammal and a second test portion comprises an antibody or affimer which, under the appropriate conditions, recognizes and binds the neutrophil specific marker NE.

The sample pad may be chromatographically connected to a test portion of the device, the test portion comprising an antibody or an antibody binding fragment thereof, affimers, aptamers or ligands or parts thereof.

When chromatographically active portions of the sample to be tested move from the sample pad towards and through the test portion, NE and CD64 may be captured onto test or control portions of the device and the remainder of the sample flowing from the sample pad is uncaptured. The uncaptured components of the test sample may be collected chromatographically into an absorbent pad, which is positioned in any orientation with respect to the test portion.

Components of the subject sample, such as red blood cells or particular white blood cells may be retained in the sample pad, for example, by selecting a pad of suitable mesh or pore size and/or by the inclusion of specific reagents such as antibodies or lectins to bind and retain these components. For example monocytes may be retained using anti-CD14 antibodies. Anti-glycophorin antibodies may be used to retain/remove red blood cells.

Once the test portion of the immunochromatographic device has been exposed to CD64 and NE in the subject sample, the method proceeds by allowing contact between the test portions and a detection binding agent. The detection marker/s may be stored in a separate portion of the kit.

The detection marker may comprise a visually detectable reporter molecule and a positive result may be essentially immediately observed in the test and/or control portions of the immunochromatographic device.

Permeabilising agents and solutions may also cause leukocyte cell lysis, fixation or solublisation and the term is used broadly herein to encompass these events, if preferred. Permeabilization is performed to facilitate access to cell markers within the cell (internal).

Permeabilization of the cells can be performed by any suitable method known in the art. These methods include, but are not limited to, exposure to a detergent (such as CHAPS, cholic acid, deoxycholic acid, digitonin, n-dodecyl-βD-maltoside, lauryl sulfate, glycodeoxycholic acid, n-lauroylsarcosine, saponin, and Triton X-100). Other permeabilising methods comprise the use of certain peptides or toxins that render membranes permeable. Permeabilization may also be performed by addition of an organic alcohol to the cells. Selection of an appropriate permeabilising agent and buffers if required can easily be performed by one of ordinary skill in the art. Permeabilization can occur concurrently with a fixation step. Fixing solutions include, for example, Cytofix/Cytoperm (BD Biosciences). Commonly used cell fixatives include, but not limited to formaldehyde, paraformaldehyde, glutaraldehyde, acetic acid, picric acid, methanol, ethanol, and acetone. Illustrative fixation buffer suitable for whole blood samples is Phosflow Lyse/Fix Buffer (BD Biosciences). Fixatives have been used for detection of both surface and intracellular antigens. Some fixatives include alcohol and formaldehyde/paraformaldehyde. Illustrative fixatives are described in U.S. Pat. No. 5,422,277 and U.S. Pat. No. 5,597,688.

In other embodiments, the detection marker may be detected using further detection protocols and devices such as will be well known to those of ordinary skill in the art. For example, colloidal metal or metal oxide particles or colloidal non-metal particles or dyes or colored latex are conveniently used.

In early experiments described in example 1 a diagnostic gate or cutoff was determined by assessing the ratio of CD64 to NNM.

As discussed above, the assay comprises:
(i) contacting the sample with an agent that permeabilises or solubilises neutrophils such that total (internal and external) amounts of CD64 are determined;
(ii) simultaneously with (i) or sequentially, contacting the sample with a binding agent that binds specifically to CD64 in the sample and forms CD64-binding agent complex a;
(iii) simultaneously with (i) and/or (ii) or sequentially, contacting the sample with a second binding agent that binds specifically to neutrophil elastase (NE) in the sample and forms NE-binding agent complex b;
(iv) employing the amount of complex a and complex b to determine the relative level of CD64 and of NE in the sample; and
(v) determining a ratio of CD64 and NE (CD64:NE) from the sample, wherein the ratio is internally corrected for the number of neutrophils in the sample determined using the level of NE in the sample and scoring the sample as comprising control or supranormal levels of neutrophil CD64/activation if the CD64:NE ratio exceeds a cut-off level predetermined as above the 95% confidence interval of the line of best fit for the CD64:NE ratio.

Thus, the method comprises measuring and determining the relative amount of each complex from steps (ii) and (iii) to obtain a modified CD64 index (ratio of CD64 to NNM) indicating or representing the average amount of CD64 per neutrophil in the sample.

The CD64 index is corrected for the number of neutrophils in the sample determined by the amount of the NE-binding agent complex measured in the sample.

In one embodiment, the assay comprises diagnosing a neonatal subject as having neonatal sepsis or having a risk of developing neonatal sepsis. Cutoff levels can be modified depending upon, inter alia, the age of the subject.

The CD64 index is compared with or plotted against the number of neutrophils in the sample determined by the amount of the NE-binding agent complex measured and the cutoff nCD64 index is corrected for the number of neutrophils in the sample. As shown in Figure 4C there is a strong correlation between total CD64 and neutrophil counts in healthy donors. However, in samples with low neutrophil counts the total amount of CD64 expressed is in fact higher. This means, surprisingly, that the cutoff nCD64 ratio indicating sepsis or risk of sepsis should not, for increased accuracy, be a constant value, but instead should be corrected on a case by case basis for the total number of neutrophils in the sample (e.g., neutrophils expressed as cells/volume). As the value of the index may be a function of the neutrophil count greater accuracy is obtained by making an adjustment of the cutoff, which was not possible when using flow cytometry alone as without a separate reference method, there is no way of getting an absolute neutrophil count by Flow cytometry. In one embodiment, a simple algorithm is embedded or available via an instrument reader to set the cutoff value for any given value of neutrophil count. , In the assay, the cutoff value is above the 95% confidence interval of the best line of fit for the CD64/NE ratio, as shown in Figure 3B. This is illustrated in Figure 5.

In one embodiment, the binding agent is immobilised on a support. Suitable supports are well known in the art, as described herein.

In some embodiments, the assay is performed wherein, in step (ii) and/or (iii), the sample is contacted with the binding agents of step (ii) and/or (iii) by applying the sample to a sample portion of an immunochromatographic or microfluidic device wherein the device sample portion is operably connected to spaced capture portions of the device and whereby the components of the sample flow from the device sample portion to and through the device capture portions, and wherein one capture portion comprises the binding agent which specifically binds to CD64 in the sample such that the CD64 is captured by the binding agent to form a binding agent-CD64 complex in the capture portion, and wherein a second capture portion comprises the binding agent which specifically binds to NE in the sample such that the NE or the binding part thereof is captured by the binding agent to form a binding agent-NE complex in the capture portion.

In one embodiment, the amount of CD64-binding agent complex and NE-binding agent complex is detected using binding agents such as antibody or antigen-binding fragments that bind to CD64 or NE respectively and directly or indirectly provide a detectable signal that can be quantified visually or photometrically including fluorometrically (by instrument reader).

In relation to this embodiment, the binding agents may be conjugated to a detectable marker or microparticles comprising a detectable marker, that provide a detectable signal. Illustrative particles and methods are described in Rundsträm, G et al. Lateral Flow Immunoassay Using Europium (III) Chelate Microparticles and Time-Resolved Fluorescence for Eosinophils and Neutrophils in Whole Blood. Clinical Chemistry 53, 342-348 (2007).

In one embodiment, the capture portion is a test line.

Illustrative binding agents include antigen-binding construct comprising a protein scaffold which is linked to one or more epitope-binding domains wherein the antigen-binding construct has at least two antigen binding sites at least one of which is from an epitope binding domain and at least one of which is from a paired VH/VL domain.

As illustrated in Example 2,NNMs, specifically NE, can provide a useful correction for the number of neutrophils, without a strong absolute correlation with neutrophil number. This is because the utility of NE is not necessarily dependent on being well correlated with neutrophil numbers, but rather being correlated with neutrophil CD64 levels and associated with neutrophil number.

Accordingly, NE may have a positive correlation with neutrophil numbers of less than about 70%, but a correlation with neutrophil-specific CD64 of not less than about 70%, and this will be effective.

By "about" is meant a measurement, quantity, level, activity, value, number, frequency, percentage, dimension, size, amount, weight or length that varies by as much as 10, 9, 8, 7, 6, 5, 4, 3, 2 or 1% to a reference measurement, quantity, level, activity, value, number, frequency, percentage, dimension, size, amount, weight or length
Each embodiment in this specification is to be applied *mutatis mutandis* to every other embodiment unless expressly stated otherwise.

In one embodiment, the present specification enables an immunoassay suitable for point of care use performed on a whole blood sample obtained from a subject, or the whole blood sample substantially depleted of one or more of red blood cells, monocytes and macrophages, for determining a modified neutrophil CD64 index.

As used herein the phrase "substantially depleted" means at least 70%, 71%, 72%, 73%, 74%, 75%, 76%, 77%, 78%, 79%, 80%, 81%, 82%, 83%, 84%, 85%, 86%, 87%, 88%, 89%, 90%,91%, 92%, 93%, 94%, 95%, 96%, 97%, 98% or 99% free of the cell, such as monocytes and macrophages.

In some embodiments, the subject is a human and includes neonatal humans. The present invention extends, however, to primates, livestock animals, laboratory test animals, companion animals and avian species as well as non-mammalian animals such as reptiles. The assay has applications, therefore in human, livestock, veterinary and wild-life therapy and diagnosis.

Whole blood refers to an essentially unprocessed sample of blood from a subject, generally anticoagulant has been added when the blood is obtained by venous collection, or it may or may not be omitted if the blood is obtained by fingerprick or fingerstick collection and used without delay at point of care.

The following kits/reagents are illustrative of the reagents used in the development of the assays described herein.

### ELISA kits used:

Item Description: Human FCGR1A/CD64 ELISA kit
Catalogue Number: LS-F4795
Company Name: LS Bio
Item Description: PMN Elastase Human ELISA Kit
Catalog Number: ab119553
Company Name: Abcam

Antibodies and recombinant proteins used:
Item Description: Neutrophil Elastase, Recombinant Protein
Catalog Number:MBS957715
Company Name: MyBioSource
Item Description: Supply Human Neutrophil Elastase
Catalog Number: INHE
Company Name: Cardinal Bioresearch
Item Description: Anti-Neutrophil Elastase antibody Mouse Monoclonal
Catalog Number: ab41179
Company Name: Abcam
Item Description: Anti-Neutrophil Elastase antibody Rabbit Polyclonal
Catalog Number: ab126154
Company Name: Abcam
Item Description: Anti-Neutrophil Elastase antibody (Biotin)
Catalog Number: ab79962
Company Name: Abcam
Item Description: Recombinant Human CD64
Catalog Number: 1257- FC
Company Name: R&D Systems
Item Description: Anti-CD64 antibody [10.1] (Biotin)
Catalog Number: ab27928
Company Name: Abcam
Item Description: Anti-CD64 antibody Rabbit Polyclonal
Catalog Number: ab95244
Company Name: Abcam
Item Description: Anti-CD64 antibody [3D3] Mouse Monoclonal
Catalog Number: ab140779

The embodiments of the kits, steps and assays are further identified with the following illustrative examples.

### Example 1

Development of an alternative to Flow cytometric based neutrophil activation assay useful for point of care (POC). Assessing the immunoassay cutoff using the ratio of CD64 to NNM.

Recognising that a POC test for measuring neutrophil activation (nCD64i) would have great clinical utility, a novel method was devised as described herein that would allow the measurement of an analogue or equivalent of the Flow Cytometry nCD64i value, using an algorithm and the values for a combination of markers that are independently suited to measurement at POC.

In some respects, the present approach draws upon some of the inventors experience in development of a lateral flow, POC test for measuring CD4 T-cells (US Patent 8,409,818 and other territories).

From the inventors experience with the CD4 POC test, it was understood that the amount of CD4 per cell is constant and therefore the amount of CD4 is directly proportional to the number of CD4 T-cells. In contrast, the amount of CD64 on neutrophils is variable (being elevated in sepsis), and the nCD64i value measured by Flow Cytometry represents the average amount of CD64 protein per neutrophil (and is sometimes expressed as Mean Fluorescence Index). Therefore nCD64i can be represented as the numerical fraction (total CD4 in blood depleted of monocytes and macrophages) / (total number of neutrophils) or CD64/neutrophil. In the Flow Cytometer, this is measured as fluorescence intensity for each individual cell and then averaged over the entire number of neutrophils sampled (typically 50,000 cells or 100,000 cells).

The inventor/s predicted that if they were able to measure the total amount of CD64 in whole blood, after removal of monocytes and macrophages, for example, using the same method shown in the example of the CD4 test, then that would give the numerator of the CD64/neutrophil fraction. This measurement of CD64 could be achieved by methods such as enzyme-linked immunosorbent assay (ELISA-type assays seldom use enzymes but are still called ELISA's) using simple laboratory facilities, or more preferably by POC methods such as lateral flow immuonochromatography following the example of CD4 T-cells in the Visitect CD4 T-cell test.

The inventor/s further recognised that the number of neutrophils, representing the denominator of the CD64/neutrophil fraction, could be measured using the same method as the CD4 T-cell test, based on a protein that demonstrates a constant or predictable level of expression for each neutrophil, such that the amount of such protein is proportional to the number of neutrophils. As one example, the neutrophil-specific protein neutrophil elastase (NE) may be measured, and is claimed in the assay methods herein. Illustrative lateral flow methods and a radioimmunoassay method for the detection and enumeration of neutrophils together with eosinophils has been reported in the literature, using the proteins eosinophil protein X (EPX) and human neutrophil lipocalin (HNL), and showing acceptable levels of agreement with traditional cell counting methods (Rundström, G et al. Lateral Flow Immunoassay Using Europium (III) Chelate Microparticles and Time-Resolved Fluorescence for Eosinophils and Neutrophils in Whole Blood. Clinical Chemistry 53, 342-348 (2007)).

Prior art methods for detecting NE are used on plasma or cell culture, where they detect the NE that has been released due to degranulation of neutrophils. In contrast, in the present assay, total NE is measured in whole blood including that inside intact neutrophils. The amount in plasma is much lower and essentially irrelevant compared to the intracellular amount.

NE has been considered as a marker for sepsis itself but only the plasma/serum amount, since the whole blood amount serves instead as a marker of neutrophil count. NE is released in response to infection but the balance between release and turnover is considered too variable to make this useful as a marker for sepsis.

It was therefore predicted that by using immunoassays such as a lateral flow immunochromatographic immunoassay for parallel measurement (in the same assay run at the same time) of both the total amount of neutrophil CD64 and the absolute number of neutrophils (for example using neutrophil elastase as the marker) in whole blood, the simple arithmetic fraction of CD64/NE could be obtained and would provide an alternative, point of care (POC) friendly measurement of the nCD64 index or (also called the nCD64 ratio). The principle of the tests for enumeration of CD4 T-cell numbers (Visitect CD4) and the herein described POC friendly nCD64i test (which may be called the SepsiTest) is shown in Figure 3.

To test the prediction that the simple arithmetic fraction of CD64/NE could be obtained as an alternative measurement of the nCD64 index, ELISAs were used to measure the total amount of Neutrophil Elastase (NE) and CD64 in whole blood from healthy volunteers. For these initial experiments (Figure 4), the monocytes and macrophages were not first removed as the total number of monocytes and macrophages is much smaller than the number of neutrophils and so the effect of these cells is not expected to be a problem, however in the final test it is expected that monocytes and macrophages will be substantially/optionally depleted.

The experimental data (see Figure 4) using ELISAs show strong correlation between the total amount of the neutrophil specific protein, neutrophil elastase (NE) and neutrophil (granulocyte) counts in healthy donors (Fig 4A; p=0.0002, R2 = 0.896).

Similarly, there is strong correlation between total CD64 and neutrophil (granulocytes) counts in healthy donors (Fig 4C, p=0.0003, R2 = 0.915). However, the surprising observation was made that the amount of CD64 per cell in healthy individuals is not constant, but instead is affected by the total number of neutrophil/granulocytes. That is, in samples with low levels of neutrophils (approaching the level of neutropenia), the total amount of CD64 is in fact higher, representing a higher amount of CD64 per cell even in these healthy individuals. To our knowledge this has not previously been observed.

The ratio between CD64 and NE therefore provides the nCD64 index, shown in Figure 4B. Because the relative amount of CD64 is higher in samples with lower total neutrophil counts, a similar relationship is seen in the calculated values for the nCD64 index, with a higher nCD64i value for samples with low neutrophil counts (R2 = 0.861). This novel observation is likely to have strong clinical significance in the development of more effective sepsis tests based on CD64 expression, because it suggests that the cutoff ratio for nCD64i should not be a constant value, as recommended in the Leuko64 Flow Cytometry kit but subject to individual optimisation in each laboratory, [Beckman Coulter - see US Patent publication no. 2013/0230867 in the name of Trillium Diagnostics LLC-this kit comprises a cocktail of three monoclonal antibodies with specificities to CD64 (FITC conjugated) and CD163 (phycoerythrin conjugated) and a proprietary fluorescent bead suspension used for instrument calibration and the standardization of the leukocyte CD64 and CD163 expression on human blood leukocytes. The kit also contain a red cell lysing solution concentrate].

Rather, the cut off should be a variable value depending on the total neutrophil count in each instant sample. An algorithm could be constructed to set the cutoff value for any given value of neutrophil count using methods well known in the art, for example setting the cutoff as above the 95% confidence interval of the line of best fit for the CD64/NE ratio shown in Figure 3B. An example of this calculation is shown in Figure 5, where the nCD64 index is calculated for a larger set of healthy control samples following monocyte depletion, and the lower and upper 95% confidence interval for the estimated nCD64 index at different amount of NE (representing neutrophil count) is shown as dotted red lines. Any value of nCD64i that is higher than the upper illustrative 95% confidence interval would therefore have a high suspicion of representing sepsis.

In some embodiments, the cutoff is set above any value between 95% and 99%. The choice of cutoff would be determined by expanded clinical studies and receiver operating characteristics (ROC) analysis.

Correction of the cutoff value in light of the observed variation in CD64 levels depending upon the neutrophil concentration may be made by various approaches known in the art. In one embodiment, the correction factor is applied to the CD64 index, such as by making it a semi-log ratio or multiplying it by an inverse of the neutrophil count). Alternatively, the cutoff numerical value is adjusted to have the same effect.

The methods for transforming the individual ELISA assays for CD64 and NE into POC tests such as lateral flow immunoassays are well known in the art, and the Visitect CD4 test and the neutrophil/eosinophil test described by Rundström et al (Rundström, G et al. Lateral Flow Immunoassay Using Europium (III) Chelate Microparticles and Time-Resolved Fluorescence for Eosinophils and Neutrophils in Whole Blood. Clinical Chemistry 53, 342-348 (2007)) are relevant examples.

For the purpose of using lateral flow immunochromatography or other POC methods to calculate the nCD64 index, it is anticipated that a simple arrangement such as that shown schematically in Figure 6 would be effective. In this predictive example, the test would have a similar format to the Visitect CD4 T-cell test, including the method for substantial removal of red blood cells, monocytes and macrophages incorporated in that test. However in place of the test line for CD4 and the reference line representing a cutoff of 350 CD4 T-cells, in one embodiment, there will be 2 test lines with one measuring the amount of CD64 and the other measuring the amount of NE or other neutrophil-specific marker. To determine a cut off, the arithmetic ratio between the measured intensity of the two lines would then be calculated, either by visual inspection or by instrument reader such as the Axxin AX-2 instrument (see for example US publication no. 2013/0162981) and the cutoff for the individual sample would be calculated from the measured amount of NE or other neutrophil-specific marker. The measured nCD64i would then be compared to the instant NE-dependent cutoff, i.e. the upper 95% confidence interval for healthy samples, and a decision on "likely sepsis" or "not likely sepsis" would be reported.

### Example 2

Further developments are described and in particular the utility of total CD64 and total NNM levels (i.e., internal and external levels) and different cutoff determinations in the diagnosis of sepsis.

In Figure 4C it is illustrated that the amount of CD64 compared to NE (the nCD64i measured by ELISA) was higher in samples with lower neutrophil/granulocyte counts, with the novel suggestion of the need for a variable cutoff for CD64i as a function of NE value to improve the use of CD64 in diagnosis of sepsis (see also Figure 5). Notably, the results in that experiment were obtained using whole blood that had been depleted of monocytes/macrophages using magnetic beads.

In further work, these observations have been extended and a simplified method is described that requires only the use of NE and CD64 values from whole blood, without the depletion of monocytes/macrophages. Monocyte/macrophage depletion is an optional step especially for samples with low values of NE where the relative contribution of monocyte/macrophage CD64 would be higher than in samples with higher values of NE, for example in patient samples having NE values of less than about 1.0 or less than about 0.5 µg/ml NE in whole blood.

In the improved method, rather than calculating the nCD64i (CD64 per neutrophil) and comparing that number with the absolute value of NE (Fig 5), the absolute value of CD64 is used in comparison with the absolute value of NE. That is, the method is derived from the work described in example 1 but provides additional insights into the system and additional strategies for using CD64 and NNM together as a sensitive diagnostic in a wide range of immunoassays. Rather than relating the amount of NNM to the absolute neutrophil/granulocyte count, the close correlation between CD64 and NNM in healthy subjects is used to define the threshold "healthy" level of CD64 for any given level of NNM.

Figure 7 shows results of ELISA for NE and CD64 in whole blood for healthy subjects (n=30), and shows that in healthy subjects, the total amount of CD64 in whole blood is closely correlated with the total amount of NE in whole blood (R2= 0.74). This is likely to reflect the correlation of NE with neutrophil/granulocyte counts, but for the purpose of our assay this correlation of NE and neutrophil/granulocyte count is not necessary - it is the NE concentration *per se* that is important. Consistent with our observations in Figure 4C but novel compared to the known art, the level of CD64 reached a plateau when NE reached around 5 µg/ml, suggesting a feedback mechanism that may limit the expression of CD64 when NE levels (and presumably neutrophil counts) are in the higher range of normal, or elevated above normal, in healthy subjects. From this figure it is predicted that sepsis patients would have elevated levels of CD64 relative to the amount of NE present in the respective patient's sample, and a presumptive cutoff for CD64 of the mean plus 2 standard deviations of the healthy samples (209 ng/ml) could be contemplated for samples with average or higher levels of NE.

Figure 8 shows the results of ELISA for NE and CD64 in whole blood for sepsis patients (red markers, n=11) together with the healthy subjects as shown in Figure 7 (blue markers). It is seen that indeed 9/11 sepsis patients have highly elevated levels of CD64 above the mean plus 2 SD. Unexpectedly however, 6/11 sepsis patients were also found to have NE levels higher than the mean plus 3 SD of healthy subjects (17.6 µg/ml), which suggests an additional cutoff based on NE levels for sepsis. This additional NE cutoff unequivocally identifies an additional sample that was just below the CD64 cutoff of 209 ng/ml.

Figure 9 shows the same results but highlighting the four different classifications of sepsis patient samples according to the cutoffs based on the mean plus 2 SD of CD64 and mean plus 3 SD of NE. By these criteria, one patient is positive only for NE, 5 patients are positive for both NE and CD64, 4 patients are positive for CD64 only, while one patient is negative for both NE and CD64, with very low values for both markers, and suggesting that the patient may have low levels of neutrophils. This result highlights that the combination of NE and CD64 is more sensitive for the diagnosis of sepsis than the use of NE or CD64 alone, probably related to the novel observation that with increasing supranormal levels of NE, the induced expression of CD64 in sepsis may be somewhat restricted by a feedback mechanism as observed in healthy subjects.

While these results suggest that the simple cutoffs for NE and CD64 would provide a highly sensitive method for diagnosis of sepsis in patients with normal or elevated levels of NE in whole blood and by inference, normal or elevated neutrophil counts, there are many patients with sepsis or at high risk of sepsis who have low neutrophil counts (neutropenia), for example many neonates and patients who have undergone chemotherapy or radiation therapy for cancer treatment.

For these neutropenic patients, advantage can be taken of the close correlation of NE versus CD64 in healthy subjects, even when the NE level is low. As shown in Figure 10, when the analysis of NE versus CD64 is limited to those healthy subjects with NE of ≤2.5 µg/ml, a very strong correlation is seen (R2 = 0.88), and the single sepsis patient not identified using the NE and CD64 cutoffs of Figures 8 and 9 (red marker) is seen to have a higher level of CD64 relative to NE than the healthy subjects (blue markers). This is further illustrated in Figure 11, where an alternative cutoff for CD64 is calculated as a multiple of the trendline for CD64 versus NE in healthy subjects. In this case, the multiple is 1.6 times the trendline and the trendline is described by the equation y = 5.6953x2 + 45.769x where y is CD64 (ng/ml) and x is NE (µg/ml). It will be obvious that this cutoff may be extrapolated until it reaches the same level of CD64 described by the simple cutoff of mean plus 2SD as in Figures 8 and 9, or higher levels as may be desired to improve the specificity of the assay for example mean plus 3 SD or higher.

On this basis, the combination of NE and CD64 markers may be used to diagnose sepsis using the algorithm of sepsis being indicated when one or more of the following conditions are met: CD64 > mean plus 2 SD of healthy subjects, OR NE > mean plus 3 SD of healthy subjects, OR CD64 > 1.6 times the level of CD64 predicted by the trendline for healthy subjects with levels of NE below 2.5 µg/ml or a higher amount such as 5 µg/ml.

However a more simple algorithm is suggested by the observation that the CD64 cutoffs can also be described by a multiple of the polynomial trendline for NE versus CD64 in healthy subjects, as shown in Figure 12A where the multiple is 1.6 and the trendline is described by the equation y = -0.0137x4 + 0.6094x3 - 9.857x2 + 65.776x, where y is CD64 (ng/ml) and x is NE (µg/ml). In this example, the red shaded area defines a "gate" of values that represent "healthy" (non-septic) levels of CD64 and NE, with an upper level of NE representing the mean plus 3 SD of healthy subjects. Figure 12B shows the sepsis patients superimposed on this model, with 11/11 (100%) sepsis patients identified correctly.

An even more simple algorithm is achieved by further extrapolation of the cutoff derived by a multiple of the trendline for CD64 versus NE. As shown in Figure 12C, extrapolation of the cutoff where the multiple is 1.6 and the trendline is described by the equation y = -0.0137x4 + 0.6094x3 - 9.857x2 + 65.776x, where y is CD64 (ng/ml) and x is NE (µg/ml) gives a similar "gate" of healthy levels of CD64 versus NE to that obtained in Figure 12B. Therefore, sepsis would be indicated in any subject where CD64 (ng/ml) > 1.6 x (-0.0137x4 + 0.6094x3 - 9.857x2 + 65.776x), where x is NE (µg/ml).

### Example 3

To better understand the utility of the herein described assay, sepsis patients and a subset of the healthy subjects were examined using the commercial Leuko64 assay, which measures the cell surface expression of CD64 on neutrophils by Flow cytometry.

The Leuko64 kit [see for example Beckman Coulter - see US Patent publication no. 2013/0230867 in the name of Trillium Diagnostics LLC] comprises a cocktail of three monoclonal antibodies with specificities to surface CD64 (FITC conjugated) and surface CD163 (phycoerythrin conjugated) and a proprietary fluorescent bead suspension used for instrument calibration and the standardization of the leukocyte CD64 and CD163 expression on human blood leukocytes.. The kit also contain a red cell lysing solution concentrate. Variants of this kit are available such as the Accellix CD64 cartridge and reader (LeukoDx)

As shown in Figure 13, Leuko64 showed highly significantly higher values for sepsis patients than for healthy controls, but using the Leuko64 kit manufacturer recommended cutoff of 1.2, only 8/11 sepsis patients were identified with this test, consistent with many reports in the literature that show around 70-80% sensitivity of this method. Similarly, detection of total whole blood CD64 alone by ELISA showed highly significantly higher values for sepsis patients than for healthy controls, but using the cutoff of mean plus 2 SD (209 ng/ml), only 9/11 sepsis patients were identified with this test. The total CD64 and NE values are determined by comparison with reference standards (standard curve) provided with commercially available kits and tested in each assay run, and then adjusted for the sample dilution used in the assays.

Individual sepsis patient samples were then examined in more detail by comparing the amount of CD64 detected by Leuko64 (surface CD64 only) versus an standard flow cytometry method in which cells were stained in the same way as for Leuko64 (surface CD64 only) or after the cells were first permeabilised using a neutrophil lysis solution comprises a detergent to allow detection of both cell surface and intracellular CD64.

Figure 14 shows the results of this analysis for patient SEP009, the patient with the lowest levels of NE in our earlier analysis. In panel A, it is shown that the neutrophils from this patient have high levels of surface expression of CD64 (red line- middle peak) consistent with very high relative CD64 expression in Leuko64 (panel B), but even higher levels of CD64 per cell when intracellular CD64 is detected as well (blue line -total -peak on right hand side). However the low level of NE (panel D) and presumed low neutrophil counts for this patient result in a total CD64 result that is below the cutoff based on CD64 alone (panel C), but detectable using the algorithm approach (Figures 8-12). This result also suggests that for patients with low levels of NE, the Leuko64 approach or other methods may be of additional utility given the very high Leuko64 result for this patient.

Figure 15 shows the same analysis for patient SEP010, the patient with elevated levels of CD64 but just below the cutoff in Figure 9 derived from the mean plus 2 SD of CD64 from healthy subjects. In this patient, there was minimal surface staining of CD64 (panel A, comparing red line and isotype control for surface staining) consistent with low relative CD64 expression in Leuko64 (panel B), but there were high levels of CD64 detected when intracellular CD64 was stained as well (panel A blue line, right hand peak). Consistent with this observation, patient SEP010 had modest levels of total CD64 by ELISA, although still just below the cutoff for total CD64 based on mean plus 2SD (panel C), whereas the Leuko64 result of 0.7 was well below the manufacturer cutoff of 1.2 for that test (panel B). However, when NE is examined the patient SEP010 is seen to have very highly elevated levels of NE, providing unequivocal diagnosis of sepsis using the cutoff derived from the mean plus 3SD of NE, and also using the cutoff derived from the trendline equation.

Figure 16 shows the same analysis for patient SEP011, a patient with elevated levels of both CD64 and NE. In this patient, there was significant surface staining of both intracellular and surface CD64 (panel A, comparing red line and isotype control for surface staining, blue line (middle peak)and isotype control for total staining) consistent with high relative CD64 expression in Leuko64 (panel B), well above the manufacturer cutoff. Patient SEP011 had highly elevated levels of total CD64 by ELISA (panel C), and also highly elevated levels of NE (panel D), providing unequivocal diagnosis of sepsis using the cutoff derived from the mean plus 3SD of NE, AND the cutoff derived from the mean plus 2SD of CD64, AND also using the cutoff derived from the trendline equation.

The results in Figures 13, 14, 15 and 16 demonstrate that a further advantage of the present method is the detection of total CD64, using ELISA or other methods and solubilized whole blood or permeabilised intact cells, rather than only cell surface CD64 as conventionally performed using Leuko64 or other methods.

The likely biological explanation for this novel observation is that only a variable proportion of CD64 is present on the surface of neutrophils at any given time. This is illustrated schematically in Figures 17 and 18. In Figure 17, modified from van der Poel 2011, in healthy individuals, low levels of CD64 (FcγR1) are expressed on the cell surface, and these are saturated by monomeric IgG which does not result in cell signaling or antigen complex internalization (Figure 17A). Upon initial stimulation of the neutrophil by interferon gamma or other stimuli associated with bacterial infection or sepsis, additional CD64 is synthesized and translocated to the surface of the neutrophil and associated with membrane microdomains, together allowing for binding of multimeric Ig in immune complexes which leads to cell signaling (Figure 17B).

However a further function of CD64 is internalization of the antigens in such immune complexes, to allow for antigen processing and presentation. As shown in Figure 18, this may result in activated neutrophils having different levels of surface CD64, even though they have the same elevated level of total CD64. On the left, a "healthy" neutrophil is shown schematically with low levels of CD64, expressed on the surface. These cells may also contain small amounts of pre-formed, intracellular CD64. In sepsis, "activated" neutrophils are shown on the right, each with equally large amounts of CD64, significantly increased compared to healthy neutrophils, but with distribution on the surface (similar to the patient sample SEP09, Figure 14), or evenly distributed between the surface and intracellular (similar to the patient sample SEP011, Figure 16), or predominantly distributed to the intracellular compartment (similar to patient sample SEP010, Figure 15). As such, any method that allows the detection of both cell surface and intracellular CD64 will allow for improved diagnosis of sepsis.

It is also noted that this method can be used to similarly detect soluble CD64 that has been shed from the surface of cells into the plasma fraction, as well as NE which has been released from activated neutrophils (sometimes known as neutrophil extracellular traps or NETs), which may be a further advantage of our method of whole blood analysis of CD64 and NE. In the known art, soluble NE and CD64 have been measured in either the serum or plasma fraction, but NOT in the whole blood fraction that would incorporate both intact cells and cells in various stages of activation and degranulation.

### REFERENCES

1. Jawad, I., Lukšić, I. & Rafnsson, S. B. Assessing available information on the burden of sepsis: global estimates of incidence, prevalence and mortality. J. Glob. Health 2, 010404 (2012).
2. Mancini, N. et al. The era of molecular and other non-culture-based methods in diagnosis of sepsis. Clin. Microbiol. Rev. 23, 235-51 (2010).
3. Wagner, T. A. et al. Emerging biomarkers for the diagnosis of severe neonatal infections applicable to low resource settings. J. Glob. Health 1, 210-23 (2011).
4. Masuda, M. & Roos, D. Association of all three types of Fc gamma R (CD64, CD32, and CD16) with a gamma-chain homodimer in cultured human monocytes. J. Immunol. 151, 7188-95 (1993).
5. Hoffmann, J. J. M. L. Neutrophil CD64: a diagnostic marker for infection and sepsis. Clin. Chem. Lab. Med. 47, 903-16 (2009).
6. van Vugt, M. J. et al. The FcgammaRIa (CD64) ligand binding chain triggers major histocompatibility complex class II antigen presentation independently of its associated FcR gamma-chain. Blood 94, 808-17 (1999).
7. Davis, B. H., Olsen, S. H., Ahmad, E. & Bigelow, N. C. Neutrophil CD64 is an improved indicator of infection or sepsis in emergency department patients. Arch. Pathol. Lab. Med. 130, 654-61 (2006).
8. Cid, J., Aguinaco, R., Sánchez, R., García-Pardo, G. & Llorente, A. Neutrophil CD64 expression as marker of bacterial infection: a systematic review and meta-analysis. J. Infect. 60, 313-9 (2010).
9. Li, S. et al. Neutrophil CD64 expression as a biomarker in the early diagnosis of bacterial infection: a meta-analysis. Int. J. Infect. Dis. 17, e12-23 (2013).
10. Du, J. et al. Diagnostic utility of neutrophil CD64 as a marker for early-onset sepsis in preterm neonates. PLoS One 9, e102647 (2014).
11. Aikaterini, Dimoula; Olivier Pradier; Zaina, Kassengera; Dyanne, Dalcomune; Turkan, Hulya; Vincent, J.-L. Serial Determinations of Neutrophil CD64 Expression for the Diagnosis and Monitoring of Sepsis in Critically Ill Patients. CID (2014). cid.oxfordjournals.org/content/58/6/820.full.
12. Rundström, G et al. Lateral Flow Immunoassay Using Europium (III) Chelate Microparticles and Time-Resolved Fluorescence for Eosinophils and Neutrophils in Whole Blood. Clinical Chemistry 53, 342-348 (2007).
13. Singer, M. et al. The Third International Consensus Definitions for Sepsis and Septic Shock (Sepsis-3). JAMA 315, 801 (2016).
14. Ausubel et al., Current Protocols in Molecular Biology, John Wiley & Sons Inc, Chapters 10 and 16, 1994.
15. Ausubel et al., Current Protocols in Molecular Biology, Supplement 47, John Wiley & Sons, New York, 1999.
16. Rose et al., A Laboratory Course Manual, Cold Spring Harbor Laboratory, Cold Spring Bate, C. A. W., and Kwiatkowski, D. (1994) Infection and Immunity 62:5261-5266.
17. Bate, C. A. W., Taverne, J., Kwiatkowski, D., and Playfair, J. H. L. (1993) Immunology 79:138-145.
18. Bate, C. A. W., Taverne, J., Bootsma, H. J., Mason, R. C. S. H., Skalko, N., Gregoriadis, G., and Playfair, J. H. L. (1992b) Immunology 76:35-41.
18. Hassan et.al. A microfluidic biochip for complete blood cell counts at the point of care. Technology 3, 201-213 (2015).19.
19. van der Poel. J. Immunol. 186(5):2699-704, 2011.

## Claims

1. An immunoassay for evaluating test sample neutrophil activation for assisting in the diagnosis of sepsis or severe infection in a subject, the assay comprising the steps of:
(i) contacting the sample with an agent that permeabilises or solubilises neutrophils such that total (internal and external) amounts of CD64 are determined;
(ii) simultaneously with (i) or sequentially, contacting the sample with a binding agent that binds specifically to CD64 in the sample and forms CD64-binding agent complex a;
(iii) simultaneously with (i) and/or (ii) or sequentially, contacting the sample with a second binding agent that binds specifically to neutrophil elastase (NE) in the sample and forms NE-binding agent complex b, wherein the amount of NE is proportional to the number of neutrophils in the sample;
(iv) employing the amount of complex a and complex b to determine the relative level of CD64 and of NE in the sample; and
(v) determining a ratio of CD64 and NE (CD64:NE) from the sample,
wherein the ratio is internally corrected for the number of neutrophils in the sample determined using the level of NE in the sample and scoring the sample as comprising control or supranormal levels of neutrophil CD64/activation if the CD64:NE ratio exceeds a cut-off level predetermined as above the 95% confidence interval of the line of best fit for the CD64:NE ratio.

2. The assay of claim 1, wherein the sample is a whole blood cell sample.

3. The assay of claim 2, wherein the sample is or has been depleted of one or more leucocytes.

4. The assay of claim 3, wherein the leukocytes are monocytes or macrophages.

5. The assay of any one of claims 1 to 4, wherein the subject is a neonatal subject.

6. The assay of any one of claims 1 to 5, wherein the assay is a point-of-care assay.

7. The assay of any one of claims 1 to 6, wherein the assay is an enzyme-linked immunosorbent (ELISA)-type, flow cytometry, bead array, lateral flow, cartridge, microfluidic or immunochromatographic based method.

8. The assay of any one of claims 1 to 7, wherein the binding agent is an antibody or an antigen-binding fragment or derivative thereof, or an antigen-binding construct.

9. The assay of claim 8, wherein the antigen-binding construct is an affimer, aptamer or a ligand or binding part thereof.

10. The assay of any one of claims 1 to 9, wherein the binding agent is immobilised on a support.

11. The assay of any one of claims 1 to 10, wherein in step (ii), the sample is contacted with the binding agents of step (ii) and (iii) by applying the sample to a sample portion of an immunoassay device wherein the device sample portion is operably connected to spaced capture portions of the device and whereby the components of the sample flow from the device sample portion to and through the device capture portions, and wherein one capture portion comprises the binding agent which specifically binds to CD64 in the sample such that the CD64 is captured by the binding agent to form a binding agent-CD64 complex in the capture portion, and wherein a second capture portion comprises the binding agent which specifically binds to NE in the sample such that the NE captured by the binding agent to form a binding agent-neutrophil number marker complex in the capture portion.

12. The assay of any one of claims 1 to 11, wherein the amount of CD64 complex and the amount of NE binding agent complex is detected using a binding agent such as an antibody or antigen-binding fragment, ligand, aptamer or affimer that binds to CD64 or NE respectively and directly or indirectly provides a detectable signal that can be quantified visually or by instrument, optionally wherein the instrument comprising software is used to input data based on the observed amounts/levels of CD64 and NE.

13. The assay of any one of claims 1 to 12, wherein the binding agent is conjugated to a detectable marker or microparticles comprising a detectable marker, that provide a detectable signal.

14. The assay of any one of claims 1 to 13, wherein the capture portion is a test line.

15. The assay of any one of claims 1 to 14, wherein the sepsis is neonatal sepsis.

## Patentansprüche

1. Immunoassay zum Auswerten der Neutrophilen-Aktivierung einer Testprobe zur Unterstützung der Diagnose einer Sepsis oder schweren Infektion bei einem Subjekt, wobei der Assay die Schritte umfasst zum
(i) Inkontaktbringen der Probe mit einem Mittel, das Neutrophile permeabilisiert oder solubilisiert, sodass eine gesamte (interne und externe) Menge von CD64 bestimmt wird;
(ii) gleichzeitig mit (i) oder nacheinander, Inkontaktbringen der Probe mit einem Bindemittel, das spezifisch an CD64 in der Probe bindet und einen CD64-Bindemittelkomplex a bildet;
(iii) gleichzeitig mit (i) und/oder (ii) oder nacheinander, Inkontaktbringen der Probe mit einem zweiten Bindemittel, das spezifisch an neutrophile Elastase (NE) in der Probe bindet und einen NE-Bindemittelkomplex b bildet, wobei die Menge an NE proportional zu der Anzahl der Neutrophilen in der Probe ist;
(iv) Verwenden der Menge an Komplex a und Komplex b, um den relativen CD64- und NE-Spiegel in der Probe zu bestimmen; und
(v) Bestimmen eines Verhältnisses von CD64 und NE (CD64:NE) aus der Probe, wobei das Verhältnis intern für die Anzahl der Neutrophilen in der Probe korrigiert wird, die unter Verwendung des NE-Spiegels in der Probe bestimmt wird, und Bewerten der Probe als Kontroll- oder übernormale Spiegel Neutrophilen-CD64/Aktivierung umfassend, wenn das CD64:NE Verhältnis einen Cut-off-Spiegel überschreitet, der als über dem 95%-Konfidenzintervall der Ausgleichsgerade für das CD64:NE Verhältnis vorbestimmt ist.

2. Assay nach Anspruch 1, wobei die Probe eine Vollblutzellprobe ist.

3. Assay nach Anspruch 2, wobei die Probe arm an einem oder mehreren Leukozyten ist oder war.

4. Assay nach Anspruch 3, wobei die Leukozyten Monozyten oder Makrophagen sind.

5. Assay nach einem der Ansprüche 1 bis 4, wobei das Subjekt ein neugeborenes Subjekt ist.

6. Assay nach einem der Ansprüche 1 bis 5, wobei der Assay ein Point-of-Care-Assay ist.

7. Assay nach einem der Ansprüche 1 bis 6, wobei der Assay ein Verfahren auf Basis des Typs eines Enzyme-linked Immunosorbent (ELISA), einer Durchflusszytometrie, einer Perlenanordung, eines Lateral Flow, einer Cartridge, einer Mikrofluidik oder einer Immunchromatographie ist.

8. Assay nach einem der Ansprüche 1 bis 7, wobei das Bindemittel ein Antikörper oder ein Antigen-bindendes Fragment oder Derivat davon oder ein Antigen-bindendes Konstrukt ist.

9. Assay nach Anspruch 8, wobei das Antigen-bindende Konstrukt ein Affimer, Aptamer oder ein Ligand oder ein bindender Teil davon ist.

10. Assay nach einem der Ansprüche 1 bis 9, wobei das Bindemittel auf einem Träger immobilisiert ist.

11. Assay nach einem der Ansprüche 1 bis 10, wobei in Schritt (ii) die Probe mit den Bindemitteln aus Schritt (ii) und (iii) durch Aufbringen der Probe auf einen Probenabschnitt einer Immunoassay-Vorrichtung in Kontakt gebracht wird, wobei der Vorrichtungsprobenabschnitt betrieblich mit beabstandeten Auffangabschnitten der Vorrichtung verbunden ist, und wodurch die Komponenten der Probe von dem Vorrichtungsprobenabschnitt zu und durch die Auffangabschnitte der Vorrichtung fließen, und wobei ein Auffangabschnitt das Bindemittel umfasst, das spezifisch an CD64 in der Probe bindet, sodass das CD64 durch das Bindemittel aufgefangen wird, um im Auffangabschnitt einen Bindemittel-CD64-Komplex zu bilden, und wobei ein zweiter Auffangabschnitt das Bindemittel umfasst, das spezifisch an NE in der Probe bindet, sodass das NE durch das Bindemittel aufgefangen wird, um in dem Auffangabschnitt einen Bindemittel-Neutrophilenzahlmarker-Komplex zu bilden.

12. Assay nach einem der Ansprüche 1 bis 11, wobei die Menge an CD64-Komplex und die Menge des NE-Bindemittelkomplexes unter Verwendung eines Bindemittels wie eines Antikörpers oder eines Antigen-bindenden Fragments, Liganden, Aptamers oder Affimers nachgewiesen wird, das an CD64 bzw. NE bindet und direkt oder indirekt ein nachweisbares Signal liefert, das visuell oder durch ein Instrument quantifiziert werden kann, wobei das Instrument optional Software umfasst, die verwendet wird, um Daten basierend auf den beobachteten Mengen/Spiegeln von CD64 und NE einzugeben.

13. Assay einem der Ansprüche 1 bis 12, wobei das Bindemittel an einen nachweisbaren Marker oder an Mikropartikel konjugiert ist, die einen nachweisbaren Marker umfassen, der ein nachweisbares Signal liefert.

14. Assay nach einem der Ansprüche 1 bis 13, wobei der Erfassungsabschnitt eine Testlinie ist.

15. Assay nach einem der Ansprüche 1 bis 14, wobei die Sepsis eine Neugeborenensepsis ist.

## Revendications

1. Dosage immunologique pour évaluer une activation de neutrophiles d'un échantillon de test pour aider au diagnostic d'une septicémie ou d'une infection grave chez un sujet, le dosage comprenant les étapes consistant à
(i) mettre l'échantillon en contact avec un agent qui perméabilise ou solubilise les neutrophiles de telle sorte que les quantités (internes et externes) totales de CD64 soient déterminées ;
(ii) simultanément avec (i) ou de manière séquentielle, mettre l'échantillon en contact avec un agent de liaison qui se lie spécifiquement au CD64 dans l'échantillon et forme un complexe a CD64-agent de liaison ;
(iii) simultanément avec (i) et/ou (ii) ou de manière séquentielle, mettre l'échantillon en contact avec un second agent de liaison qui se lie spécifiquement à l'élastase de neutrophiles (NE) dans l'échantillon et forme un complexe b NE-agent de liaison, dans lequel la quantité de NE est proportionnelle au nombre de neutrophiles dans l'échantillon ;
(iv) utiliser la quantité de complexe a et de complexe b pour déterminer le taux relatif de CD64 et de NE dans l'échantillon ; et
(v) déterminer un rapport de CD64 et NE (CD64:NE) à partir de l'échantillon, dans lequel le rapport est corrigé en interne pour le nombre de neutrophiles dans l'échantillon déterminé en utilisant le taux de NE dans l'échantillon et en notant l'échantillon comme comprenant des niveaux de contrôle ou supranormaux de CD64/d'activation de neutrophiles si le rapport CD64:NE dépasse un seuil d'inclusion prédéterminé comme étant supérieur à l'intervalle de confiance de 95 % de la ligne de meilleur ajustement pour le rapport CD64:NE.

2. Dosage selon la revendication 1, dans lequel l'échantillon est un échantillon de cellules sanguines entières.

3. Dosage selon la revendication 2, dans lequel l'échantillon est, ou a été, appauvri d'un ou de plusieurs leucocytes.

4. Dosage selon la revendication 3, dans lequel les leucocytes sont des monocytes ou des macrophages.

5. Dosage selon l'une quelconque des revendications 1 à 4, dans lequel le sujet est un sujet néonatal.

6. Dosage selon l'une quelconque des revendications 1 à 5, dans lequel le dosage est un dosage au point d'intervention.

7. Dosage selon l'une quelconque des revendications 1 à 6, dans lequel le dosage est un procédé de type immuno-absorption-enzymatique (ELISA), de cytométrie de flux, de réseau de billes, de flux latéral, de cartouche, microfluidique ou immunochromatographique.

8. Dosage selon l'une quelconque des revendications 1 à 7, dans lequel l'agent de liaison est un anticorps ou un fragment de liaison à l'antigène ou un dérivé de celui-ci, ou une construction de liaison à l'antigène.

9. Dosage selon la revendication 8, dans lequel la construction de liaison à l'antigène est un affimère, un aptamère ou un ligand ou une partie de liaison de ceux-ci.

10. Dosage selon l'une quelconque des revendications 1 à 9, dans lequel l'agent de liaison est immobilisé sur un support.

11. Dosage selon l'une quelconque des revendications 1 à 10, dans lequel, à l'étape (ii), l'échantillon est mis en contact avec les agents de liaison des étapes (ii) et (iii) en appliquant l'échantillon à une partie d'échantillon d'un dispositif de dosage immunologique, dans lequel la partie d'échantillon de dispositif est raccordée de manière fonctionnelle à des parties de capture espacées du dispositif et selon lequel les composants de l'échantillon s'écoulent de la partie d'échantillon de dispositif vers, et à travers, les parties de capture de dispositif, et dans lequel une partie de capture comprend l'agent de liaison qui se lie spécifiquement à CD64 dans l'échantillon de telle sorte que le CD64 soit capturé par l'agent de liaison pour former un complexe agent de liaison-CD64 dans la partie de capture, et dans lequel une seconde partie de capture comprend l'agent de liaison qui se lie spécifiquement à NE dans l'échantillon de telle sorte que NE soit capturé par l'agent de liaison pour former un complexe agent de liaison-marqueur du nombre de neutrophiles dans la partie de capture.

12. Dosage selon l'une quelconque des revendications 1 à 11, dans lequel la quantité de complexe CD64 et la quantité de complexe NE-agent de liaison sont détectées à l'aide d'un agent de liaison tel qu'un anticorps ou un fragment de liaison à l'antigène, un ligand, un aptamère ou un affimère qui se lie respectivement à CD64 ou NE et fournit, directement ou indirectement, un signal détectable qui peut être quantifié visuellement ou par un instrument, éventuellement dans lequel l'instrument comprenant un logiciel est utilisé pour saisir des données basées sur les quantités/niveaux observés de CD64 et NE.

13. Dosage selon l'une quelconque des revendications 1 à 12, dans lequel l'agent de liaison est conjugué à un marqueur détectable ou à des microparticules comprenant un marqueur détectable, qui fournissent un signal détectable.

14. Dosage selon l'une quelconque des revendications 1 à 13, dans lequel la partie de capture est une ligne de test.

15. Dosage selon l'une quelconque des revendications 1 à 14, dans lequel la septicémie est une septicémie néonatale.
